(19) Europäisches Patentamt
European Patent Office
Office européen des brevets

(11) **EP 2 371 907 B1**

(12) **EUROPÄISCHE PATENTSCHRIFT**

(45) Veröffentlichungstag und Bekanntmachung des Hinweises auf die Patenterteilung:
**25.09.2019 Patentblatt 2019/39**

(21) Anmeldenummer: **11171703.9**

(22) Anmeldetag: **25.11.2008**

(51) Int Cl.:
*C09C 1/00* (2006.01)      *A61K 8/02* (2006.01)
*A61K 8/19* (2006.01)      *C09D 5/36* (2006.01)
*C08K 9/02* (2006.01)
*A61K 8/25* (2006.01)      *A61Q 1/02* (2006.01)
*A61Q 1/06* (2006.01)      *A61Q 1/04* (2006.01)
*A61Q 1/10* (2006.01)      *A61Q 1/08* (2006.01)
*A61Q 3/02* (2006.01)      *A61Q 1/12* (2006.01)
*A61Q 19/00* (2006.01)     *A61Q 13/00* (2006.01)
*A61Q 17/04* (2006.01)     *A61Q 19/10* (2006.01)

(54) **Effektpigmente basierend auf künstlich hergestellten Substraten mit enger Größenverteilung**

Effect pigment based on artificially produced substrates with dense particle size distribution

Pigments à effet à base de substrats fabriqués de manière artificielle ayant une répartition précise de grandeurs

(84) Benannte Vertragsstaaten:
**AT BE BG CH CY CZ DE DK EE ES FI FR GB GR HR HU IE IS IT LI LT LU LV MC MT NL NO PL PT RO SE SI SK TR**

(30) Priorität: **20.02.2008 EP 08003114**

(43) Veröffentlichungstag der Anmeldung:
**05.10.2011 Patentblatt 2011/40**

(62) Dokumentnummer(n) der früheren Anmeldung(en) nach Art. 76 EPÜ:
**08872586.6 / 2 217 664**

(73) Patentinhaber: **Eckart GmbH**
**91235 Hartenstein (DE)**

(72) Erfinder:
• **Kaupp, Günter**
  **91284 Neuhaus (DE)**
• **Schmidt, Ulrich**
  **91217 Hersbruck (DE)**
• **Schumacher, Dirk**
  **91257 Pegnitz (DE)**
• **Schneider, Ralph**
  **91207 Lauf (DE)**

(74) Vertreter: **Walcher, Armin et al**
**Louis, Pöhlau, Lohrentz**
**Patentanwälte**
**Postfach 30 55**
**90014 Nürnberg (DE)**

(56) Entgegenhaltungen:
**EP-A1- 2 009 066        WO-A-2006/110359**
**US-A1- 2006 042 509**

**Beschreibung**

Die vorliegende Erfindung betrifft neue, auf künstlichen Substraten basierende Effektpigmente mit verbesserten optischen Eigenschaften.

Außerdem betrifft die vorliegende Erfindung die Verwendung dieser Effektpigmente in Kosmetika, Farben, Druckfarben, Lacken, Pulverlacken, Elektrotauchlacken und Kunststoffen.

Die optische Wirkung von Effektpigmenten beruht auf der gerichteten Reflexion von Licht an überwiegend flächig ausgebildeten, zueinander im Wesentlichen parallel ausgerichteten lichtbrechenden Pigmentteilchen. Diese Pigmentteilchen weisen in der Regel ein im Wesentlichen transparentes Substrat und eine oder mehrere Beschichtungen auf dem Substrat auf. Je nach Zusammensetzung der Beschichtung(en) der Pigmentteilchen erzeugen Interferenz-, Reflexions- und Absorptionsphänomene Farb- und Helligkeitseindrücke. Unregelmäßigkeiten in der zu beschichtenden Substratoberfläche oder farbige Verunreinigungen des Substrats können zu unerwünschten Streulichteffekten oder Farbunreinheiten im Endprodukt führen. Insbesondere bei Verwendung natürlicher Substratmaterialien, wie natürlichem Glimmer, treten diese unerwünschten Streulichteffekte und/oder farblichen Verunreinigungen auf. Künstliche Substrate bieten unter Umgehung genannter Nachteile neue Möglichkeiten für qualitativ hochwertige Pigmente mit neuen Farb- und Glanzeffekten.

In dem US Patent 3,331 ,699 A werden Perlglanzpigmente basierend auf Glasplättchen mit Interferenzfarben und intensivem Glitzereffekt beschrieben. Die Glasplättchen sind mit einer durchscheinenden hochbrechenden Metalloxidschicht beschichtet. Die Farbe der Pigmente ist abhängig vom gewählten Metalloxid und der Dicke der Metalloxidschicht.

Metallbeschichtete Glasplättchen, die gegen korrosive Einflüsse mit einer zusätzlichen Schutzschicht aus Metalloxiden wie $SiO_2$, $Al_2O_3$ oder $TiO_2$ belegt sind, werden in dem US-Patent 5,436,077 A beschrieben. In ihrem Aussehen sind diese beschichteten Glasplättchen vergleichbar mit Metallfolien.

Mit Titan- und/ oder Eisenoxiden beschichtete Glasplättchen sind aus dem US-Patent 6,045,914 A bekannt. Die durchschnittliche Teilchengröße der Glasplättchen liegt in einem Bereich von ungefähr 1 bis 250 $\mu m$, die Dicke bei ca. 0,1 bis 10 $\mu m$.

In der WO 2004/055119 A1 werden Interferenzpigmente auf der Basis von beschichteten plättchenförmigen Substraten beschrieben. Die Substrate sind hierbei mit einer ersten Schicht aus $SiO_2$ belegt, auf die anschließend eine hochbrechende Schicht, bestehend aus beispielsweise $TiO_2$, $ZrO_2$, $SnO_2$, $Cr_2O3$, $Fe_2O_3$ oder $Fe_3O_4$ bzw. ein Interferenzsystem aus alternierenden hoch- und niedrigbrechenden Schichten aufgebracht ist. Optional können die Pigmente noch eine äußere Schutzschicht aufweisen.

Thermisch und mechanisch stabile metalloxidbeschichtete Effektpigmente basierend auf dünnen Glasplättchen mit einer Dicke $\leq 1,0$ $\mu m$ sind aus der WO 2002/090448 A2 bekannt.

Die optischen Eigenschaften von Effektpigmenten können nach der WO 2006/110359 A2 durch eine geeignete Partikelgrößenverteilung beeinflusst werden. Die hier beschriebenen klassierten metalloxidbeschichteten Glasplättchen weisen einen $D_{10}$ von wenigstens 9,5 $\mu m$, vorzugsweise von 9,5 $\mu m$, auf. Nachteilig ist, dass die Pigmente einen Größenbereich mit einem $D_{90}$-Wert von maximal 85 $\mu m$, vorzugsweise von etwa 45 $\mu m$, aufzuweisen haben. Mithin können gemäß der Lehre der WO 2006/110359 A2 nur kleinteilige Effektpigmente mit verbessertem Glitzereffekt bereitgestellt werden.

Anwendungstechnische Nachteile, wie beispielsweise die Verstopfung von Filtern, können gemäß der Lehre der WO 2007/114442 A1 durch eine geeignete Partikelgrößenverteilung vermieden werden, wobei der $D_{10}$ zwischen 4,7 und 25 $\mu m$ und das Verhältnis von $D_{90}$ zu $D_{10}$ zwischen 2 und 3 liegt.

US 2006/0042509 A1 offenbart mit $SiO_2$ und Ceroxid beschichtete Perlglanzpigmente. EP 2 009 066 A1 betrifft flockenförmige Partikel und Glanzpigmente. WO 97/29059 A1 betrifft verbesserte Perlglanzpigmente zur Außenanwendung. US 5,156,889 offenbart ein Verfahren zur Stabilisierung von metalloxidbeschichteten Glimmerpigmenten gegen Licht und Feuchtigkeit.

Bei den bislang im Stand der Technik bekannten Effektpigmenten ist nachteilig, dass diese nur eine geringe Farbreinheit aufweisen. So werden von einem Betrachter einer Mehrzahl an Effektpigmenten einer Charge unter dem Lichtmikroskop Effektpigmente mit verschiedenen, d.h. voneinander unterschiedlichen Farben, wahrgenommen.

Der vorliegenden Erfindung liegt die Aufgabe zugrunde, neue Effektpigmente mit verbesserten optischen Eigenschaften, insbesondere mit einer gegenüber dem Stand der Technik erhöhten Farbreinheit bei einem konstanten Lichteinfalls- und Beobachtungswinkel, bereitzustellen. Weiterhin ist es Aufgabe der Erfindung, ein Verfahren zur Herstellung dieser Effektpigmente zur Verfügung zu stellen.

Die Aufgabe wurde gelöst durch Bereitstellung von Effektpigmenten gemäß Anspruch 1.

Bevorzugte Weiterbildungen sind in den abhängigen Ansprüchen 2 bis 14 angegeben.

Die erfindungsgemäßen Effektpigmente sind erhältlich mittels eines Verfahrens zur Herstellung, welches folgende Schritte umfasst:

a) Größenklassieren der künstlichen Substrate,

b) Beschichten der künstlichen Substrate.

Vorzugsweise erfolgt das Beschichten der Substrate in Schritt b) nach dem Größenklassieren in Schritt a).

Des Weiteren ist Gegenstand der Erfindung die Verwendung der erfindungsgemäßen Effektpigmente in Kosmetika, Kunststoffen und Beschichtungszusammensetzungen, wie Farben, Druckfarben, Lacken, Pulverlacken und Elektrotauchlacken.

Unter Effektpigmenten mit verbesserten optischen Eigenschaften werden im Sinne dieser Erfindung Effektpigmente verstanden, die sich insbesondere durch ihre hohe Farbreinheit bei einem konstanten Lichteinfalls- und Beobachtungswinkel auszeichnen. Die erfindungsgemäßen Effektpigmente weisen mithin in ihrer jeweiligen Gesamtheit eine einheitliche Farbe bzw. einen einheitlichen Farbton auf. Somit kommt es für einen Betrachter zu keinen merklichen farblichen Unterschieden von einem erfindungsgemäßen Effektpigment zu einem nächsten erfindungsgemäßen Effektpigment in einer Gesamtheit.

Überraschenderweise wurde gefunden, dass Substrate mit engem Span in der Teilchengrößenverteilung, die mit wenigstens einer optisch wirksamen Beschichtung beschichtet sind, eine deutlich höhere Farbreinheit gegenüber Effektpigmenten mit einem breiteren Span, wie dies im Stand der Technik der Fall ist, aufweisen.

Zur Charakterisierung der Teilchengrößenverteilung wird erfindungsgemäß der Span $\Delta D$, definiert als $\Delta D = (D_{90} - D_{10})/D_{50}$, verwendet. Je kleiner der Span ist, desto enger ist die Teilchengrößenverteilung.

Der $D_{10}$-Wert gibt den Wert der Längsausdehnung der Effektpigmente an, der mittels Lasergranulometrie als Kugeläquivalent ermittelt wird, den 10% der Pigmentteilchen der Gesamtheit aller Teilchen unterschreiten oder maximal aufweisen. Der $D_{50}$-Weli bzw. der $D_{90}$-Wert geben entsprechend die maximalen Längsausdehnungen der Effektpigmente an, die mittels Lasergranulometrie als Kugeläquivalente ermittelt werden, welche 50 % bzw. 90 % der Teilchen der Gesamtheit aller Teilchen unterschreiten oder maximal aufweisen.

Die erfindungsgemäßen Effektpigmente besitzen einen Span $\Delta D$ in einem Bereich von 0,7 bis 1,4, vorzugsweise von 0,7 bis 1,3, weiterhin bevorzugt von 0,8 bis 1,2, besonders bevorzugt von 0,8 bis 1,1.

Oberhalb eines Spans $\Delta D$ von 1,4 werden keine farbreinen Effektpigmente erhalten. Effektpigmente unterhalb eines Spans der Größenverteilung von 0,7 können im Rahmen der üblichen Methoden nur sehr aufwändig und damit nicht mehr wirtschaftlich hergestellt werden.

Die erfindungsgemäßen Effektpigmente weisen die in Tabelle 2 angegebenen Kombinationen von $D_{90}$-, $D_{50}$- und $D_{10}$-Werten auf. Hierbei werden die $D_{10}$, $D_{50}$ und $D_{90}$-Werte der Tabelle 2 nur in der Art kombiniert, daß sich ein Span $\Delta D$ von 0,7-1,4 ergibt. Kombinationen von $D_{10}$-, $D_{50}$- und $D_{90}$-Werten, die zu einem Span führen, der nicht im Bereich $\Delta D$ von 0,7 -1,4 liegt, sind keine erfindungsgemäßen Ausführungsformen.

Tabelle 2: Kombination von Wertbereichen der $D_{90}$-, $D_{50}$- und $D_{10}$-Werte

| $D_{90}$ ($\mu$m) | $D_{50}$ ($\mu$m) | $D_{10}$ ($\mu$m) |
|---|---|---|
| 20 - 45 | 10 - 35 | 5 - 25 |
| 70 - 110 | 30 - 65 | 20 - 45 |
| 120 - 180 | 40 - 140 | 25 - 65 |
| 400 - 490 | 135 - 250 | 75 - 110 |

Es hat sich überraschend gezeigt, dass die in Tabelle 2 angegebenen Kombinationen an Wertebereichen der $D_{90}$-, $D_{50}$- und $D_{10}$-Werte äußerst farbreine Effektpigmente ergeben.

Dabei hat sich überraschenderweise gezeigt, dass die absolute Größe der Effektpigmente nicht entscheidend ist, sondern das Verhältnis der Größen zueinander. Entscheidend ist, dass der Span $\Delta D = (D_{90} - D_{10})/D_{50}$ in einem engen Bereich von 0,7 bis 1,4 liegt. Dabei können beispielsweise die $D_{50}$-Werte der Effektpigmente bei 15, 20, 25, 30 $\mu$m oder auch bei 50, 80, 100, 150, 200 oder 250 $\mu$m liegen. Es hat sich gezeigt, dass überraschenderweise auch bei einer Mehrzahl von größeren Effektpigmenten farbreine Effektpigmente erhalten werden, wenn der Span $\Delta D$ in einem Bereich von 0,7 bis 1,4 liegt.

Die mittlere Dicke der künstlichen plättchenförmigen Substrate beträgt 500 nm bis 2.000 nm und bevorzugt 750 bis 1.500 nm.

Das Aspektverhältnis der erfindungsgemäßen Effektpigmente, berechnet aus dem Quotient von Teilchendurchmesser zu Dicke, liegt vorzugsweise bei 2- 980, bevorzugt bei 5 - 950 und besonders bevorzugt bei 10 - 920.

Beschichtet man Substrate unterhalb einer mittleren Dicke von 500 nm mit hochbrechenden Metalloxiden, so hat das Substrat einen deutlichen optischen Einfluss auf die Interferenzfarbe des Gesamtsystems. Daher erhält man Effektpigmente, die nicht mehr die gewünschte hohe Farbreinheit aufweisen. Außerdem nimmt die mechanische Stabilität dieser Effektpigmente beispielsweise gegenüber Scherkräften deutlich ab. Zudem dauern die Beschichtungszeiten dieser dünnen Substrate mit beispielsweise hochbrechenden Metalloxiden oder semitransparenten Metallen aufgrund der hohen spezifischen Oberflächen dieser Pigmente sehr lange, was hohe Herstellkosten verursacht.

Oberhalb einer mittleren Substratschichtdicke von 2.000 nm werden die Effektpigmente insgesamt zu dick. Damit einher geht eine schlechtere Deckfähigkeit sowie eine geringere planparallele Orientierung im Anwendungsmedium. Aus der schlechteren Orientierung wiederum resultiert ein verminderter Glanz.

Bei einer bevorzugten Ausführungsform beträgt die Standardabweichung der Dicke der künstlichen Substrate 15 % bis 100 % und besonders bevorzugt 20 % bis 70 %.

Die mittlere Dicke wird anhand eines gehärteten Lackfilmes, in dem die Effektpigmente im Wesentlichen planparallel zum Untergrund ausgerichtet sind, bestimmt. Hierzu wird ein Querschliff des gehärteten Lackfilmes unter einem Rasterelektronenmikroskop (REM) untersucht, wobei die Dicke von 100 Effektpigmenten bestimmt und statistisch gemittelt wird.

Unterhalb einer Standardabweichung von 15 % werden farbflopende Effektpigmente erhalten. Oberhalb einer Standardabweichung von 100 % sind derart viele dickere Pigmente in dem gesamten Pigmentensemble enthalten, dass es dann zu schlechterer Orientierung und damit zu Glanzverlusten kommt.

Es hat sich überraschend gezeigt, dass Effektpigmente, deren künstliche Substrate eine mittlere Dicke von 500 bis 2000 nm und zugleich einen Span $\Delta D$ von 0,7 bis 1,4 aufweisen, die gewünschte außerordentliche Farbreinheit bei einem konstanten Lichteinfalls- und Beobachtungswinkel aufweisen.

Bei den Effektpigmenten handelt es sich vorzugsweise um Perlglanzpigmente oder um optisch variable Pigmente.

Die erfindungsgemäßen Effektpigmente können hierbei auch in Mischung mit weiteren Effektpigmenten vorliegen.

Effektpigmente auf der Basis von natürlichem Glimmer sind seit langem bekannt. Allerdings ist bei der Verwendung natürlichen Glimmers als Substrat zu beachten, dass dessen Oberfläche nicht ideal glatt ist, sondern Unregelmäßigkeiten, wie z.B. Stufen aufweist. Diese Unregelmäßigkeiten limitieren die Qualität der resultierenden Effektpigmente, da sie oft zu unterschiedlichen Farben innerhalb desselben Pigmentplättchens führen. Hierdurch wird insbesondere die Farbreinheit erheblich vermindert. Des weiteren kann durch im natürlichen Glimmer enthaltenen Verunreinigungen an Fremdionen der Farbeindruck der Effektpigmente verändert sein, so dass eine Mehrzahl von Effektpigmenten, die aus einer Produktionscharge stammen, voneinander verschiedene Farben oder Farbtöne aufweisen, mithin die Pigmentmischung eine geringe Farbreinheit aufweist.

Umgangen werden können diese Nachteile natürlichen Glimmers durch die Verwendung künstlicher Substrate. Unter künstlichen Substraten werden Substrate verstanden, die als solche in der Natur nicht vorkommen, sondern synthetisiert werden müssen. Geeignete Basissubstrate für die erfindungsgemäßen Effektpigmente sind beispielsweise synthetische, nichtmetallische, plättchenförmige Substrate. Die Substrate sind vorzugsweise im Wesentlichen transparent, bevorzugt transparent.

Gemäß einer bevorzugten Variante der Erfindung werden als Substrate Glasplättchen, Plättchen aus synthetischem Glimmer, $SiO_2$-Plättchen, Polymerplättchen, plättchenförmiges Bismuthoxychlorid und/oder plättchenförmige Aluminiumoxide oder Gemische davon verwendet. Bevorzugt werden für die erfindungsgemäßen Effektpigmente Substrate bestehend aus Glasplättchen oder synthetischem Glimmer eingesetzt.

Die erfindungsgemäßen Effektpigmente können Substrate sein, die mit einer oder mehreren optisch wirksamen Schichten oder Beschichtungen versehen sind. Unter einer optisch wirksamen Schicht oder Beschichtung wird eine Schicht bzw. eine Beschichtung verstanden, an der einfallendes Licht durch physikalische Effekte wie Reflexion, Interferenz, Absorption, Lichtbrechung, etc. wahrnehmbare Farbeffekte erzeugt.

Als optische wirksame Schichten oder Beschichtungen werden vorzugsweise Schichten aufgebracht, die Metalloxide, Metalloxidhydrate, Metallsuboxide, Metalle, Metallfluoride, Metallnitride, Metalloxynitride oder Mischungen davon umfassen. Gemäß einer bevorzugten Variante bestehen die optisch wirksamen Schichten oder Beschichtungen aus den vorstehend genannten Materialien,

Die Begriffe Schichten oder Beschichtungen werden im Sinne dieser Erfindung austauschbar verwendet, sofern nicht anders angegeben.

Gemäß einer bevorzugten Variante der Erfindung kann eine Beschichtung oder können mehrere Beschichtungen einen Brechungsindex $n \geq 1,9$ aufweisen, mithin hochbrechend sein.

Wenn die erfindungsgemäßen Effektpigmente mehrere Schichten aufweisen, sind diese in Bezug auf den Brechungsindex vorzugsweise alternierend angeordnet, so das auf eine hoch brechende Schicht mit einem Brechungsindex von $n \geq 1,9$ vorzugsweise eine niedrigbrechende Schicht mit einem Brechungsindex $n < 1,9$ folgt bzw. umgekehrt.

Die Beschichtung kann dabei entweder das gesamte Substrat umhüllen oder nur partiell auf dem Substrat vorliegen. Die Metalloxid-, Metalloxidhydrat-, Metallsuboxid-, Metall-, Metallfluorid-, Metallnitrid-, Metalloxynitridschichten oder Schichten mit Mischungen von den vorgenannten Verbindungen können niedrigbrechend (Brechungsindex $< 1,9$) oder hochbrechend (Brechungsindex $n \geq 1,9$) sein.

Bei einer bevorzugten Ausführungsform umfasst oder ist das Substrat ein niedrig brechendes plättchenförmiges Substrat und die Beschichtung weist mindestens eine hochbrechende Schicht ($n \geq 1{,}9$) auf. Vorzugsweise werden als Substratplättchen Glasplättchen, synthetische Glimmerplättchen und/oder $SiO_2$-Plättchen verwendet.

Als Metalloxide und/oder Metalloxidhydrate werden vorzugsweise Titanoxid, Titandioxid, Titanoxidhydrat, Aluminiumoxid, Aluminiumoxidhydrat, Siliziumoxid, Siliziumdioxid, Eisenoxid, Eisenhydroxid, Zinnoxid, Chromoxid, Antimonoxid, Ceroxid oder deren Mischoxide verwendet.

Bei einer weiteren erfindungsgemäßen Ausgestaltung der Erfindung sind die Substrate mit Metallschichten beschichtet. Dabei werden bevorzugt semitransparente Metallschichten aufgebracht, um Effektpigmente mit Interferenzfarben nach dem Fabry-Perot-Effekt bereitzustellen.

Geeignete Metalle zur Erzeugung der semitransparenten Metallschichten sind beispielsweise Aluminium, Chrom, Nickel, Silber, Gold, Titan, Kupfer oder deren Legierungen.

Die Schichtdicke der semitransparenten Metallschichten liegt bevorzugt in einem Bereich von 4 bis 40 nm und besonders bevorzugt 5 bis 30 nm.

Als Metallfluorid wird gemäß einer Variante Magnesiumfluorid als Beschichtung aufgebracht. Als Metallnitride oder Metalloxynitride können beispielsweise die der Metalle Titan, Zirkonium und/oder Tantal eingesetzt werden.

Bevorzugt wird das Substrat mit Metalloxid- und/oder Metalloxidhydratschichten beschichtet.

Beispiele niedrigbrechender Schichten umfassen Aluminiumoxid, Aluminiumoxidhydrat, Siliziumoxid, Siliziumoxidhydrat und/oder Magnesiumfluorid.

Als hochbrechende Schichten werden beispielsweise Titandioxid, Titansuboxide, Eisenoxid, Eisenhydroxid, Zinnoxid, Zinkoxid, Zirkoniumoxid, Ceroxid, Cobaltoxid, Chromoxid, Antimonoxid oder deren Mischoxide eingesetzt.

Unterschiedliche Farben bei den erfindungsgemäßen Effektpigmenten sind hierbei durch Wahl des Schichtmaterials und der Schichtdicke einstellbar, wie Tabelle 1 beispielhaft zu entnehmen ist.

| | Belegung/ Schichtdicke | Farbe |
|---|---|---|
| Silberweiße Perlglanzpigmente | $TiO_2$: 40 - 60 nm | silber |
| Interferenzpigmente | $TiO_2$: 60 - 80 nm | gelb |
| | $TiO_2$: 80 - 100 nm | rot |
| | $TiO_2$: 100 - 140 nm | blau |
| | $TiO_2$: 120 - 160 nm | grün |
| | $TiO_2$: 280 - 320 nm | Grün (III. Ordnung) |
| Farbglanzpigmente | $Fe_2O_3$: 35 - 45 nm | bronze |
| | $Fe_2O_3$: 45 - 55 nm | kupfer |
| | $Fe_2O_3$: 55 - 65 nm | rot |
| | $Fe_2O_3$: 65 - 75 nm | rotviolett |
| | $Fe_2O_3$: 75 - 85 nm | rotgrün |
| | $Fe_3O_4$ | schwarz |
| Kombinationspigmente | $TiO_2$/ $Fe_2O_3$ | Goldtöne |
| | $TiO_2$/ $Cr_2O_3$ | grün |
| | $TiO_2$/ Berliner Blau | tiefblau |

Weisen die erfindungsgemäßen Effektpigmente eine Beschichtung mit Titandioxid auf, kann das Titandioxid in der Rutil- oder Anataskristallmodifikation vorliegen. Die qualitativ besten und stabilsten Perlglanzpigmente werden erhalten, wenn die Titandioxidschicht in der Rutilform vorliegt. Die Rutilform kann erhalten werden, indem beispielsweise vor Aufbringung der Titandioxidschicht eine Schicht aus $SnO_2$ auf das Substrat oder das Pigment aufgebracht wird. Auf einer Schicht aus $SnO_2$ kristallisiert $TiO_2$ bei Aufbringung in der Rutilmodifikation.

Die Dicke der Metalloxid-, Metalloxidhydrat-, Metallsuboxid-, Metall-, Metallfluorid- oder Metallnitridschichten oder von Schichten mit einer Mischung von den vorgenannten Verbindungen liegt üblicherweise in einem Bereich von 30 - 350 nm, weiter bevorzugt von 50 - 300 nm.

Die Metalloxid-, Metalloxidhydrat-, Metallsuboxid-, Metall-, Metallfluorid- und/oder Metallnitridschichten können Farbmittel wie Berliner Blau, Ultramarin, Karminrot, Azopigmenten, Phthalocyaninen oder FD & C Farbstoffen/-lacken enthalten oder aufweisen. Hierbei können die Farbmittel in der Beschichtung enthalten sein und/oder auf dieser Beschichtung aufgebracht und gegebenenfalls durch Haftvermittler auf diesen fixiert sein.

Um die erfindungsgemäßen Effektpigmente besser vor Witterungseinflüssen zu schützen, sind diese zusätzlich mit einer äußeren Schutzschicht belegt. Diese umfaßt oder besteht bevorzugt aus einer oder zwei Metalloxidschichten der Elemente Si, Al oder Ce. Besonders bevorzugt ist hierbei eine Reihenfolge, bei der zunächst eine Ceroxidschicht aufgebracht ist, der dann eine $SiO_2$-Schicht folgt, wie in der WO 2006/021386 A1 beschrieben, die hiermit unter Bezugnahme aufgenommen ist.

Die äußere Schutzschicht kann des Weiteren an der Oberfläche organisch-chemisch modifiziert sein. Beispielsweise können ein oder mehrere Silane auf dieser äußeren Schutzschicht aufgebracht sein. Bei den Silanen kann es sich um Alkylsilane mit verzweigtkettigen oder unverzweigten Alkylresten mit 1 bis 24 C-Atomen, vorzugsweise 6 bis 18 C-Atomen handeln.

Bei den Silanen kann es sich aber auch um organofunktionelle Silane handeln, die eine chemische Anbindung an einen Kunststoff, ein Bindemittel eines Lackes oder einer Farbe, etc. ermöglichen.

Die vorzugsweise als Oberflächenmodifizierungsmittel verwendeten organofunktionellen Silane, die geeignete funktionelle Gruppen aufweisen, sind kommerziell verfügbar und werden beispielsweise von der Fa. Degussa, Rheinfelden, Deutschland, hergestellt und unter dem Handelsnamen "Dynasylan®" vertrieben. Weitere Produkte können von der Fa. OSi Specialties (Silquest®-Silane) oder von der Fa. Wacker, beispielsweise Standard- und α-Silane aus der GENIOSIL®-Produktgruppe, bezogen werden.

Beispiele hierfür sind 3-Methacryloxypropyltrimethoxysilan (Dynasylan MEMO, Silquest A-174NT), Vinyltri(m)ethoxysilan (Dynasylan VTMO bzw. VTEO, Silquest A-151 bzw. A-171), 3-Mercaptopropyltri(m)ethoxysilan (Dynasylan MTMO oder 3201; Silquest A-189), 3-Glycidoxypropyltrimethoxysilan (Dynasylan GLYMO, Silquest A-187), tris-(3-Trimethoxysilylpropyl)isocyanurat (Silquest Y-11597), gamma-Mercaptopropyltrimethoxysilan (Silquest A-189), Bis-(3-Triethoxysilylpropyl)polysulfid (Silquest A-1289), Bis-(3-Triethoxysilyl)disulfid (Silquest A-1589), beta-(3,4-Epoxycyclohexyl)ethyltrimethoxysilan (Silquest A-186), Bis(triethoxysilyl)ethan (Silquest Y-9805), gamma-Isocyanatopropyltrimethoxysilan (Silquest A-Link 35, GENIOSIL GF40), (Methacryloxymethyl)tri(m)ethoxysilan (GENIOSIL XL 33, XL 36), (Methacryloxymethyl)(m)ethyldimethoxysilan (GENIOSIL XL 32, XL 34), Isocyanatomethyl)trimethoxysilan (GENIOSIL XL 43), (Isocyanatomethyl)methyldimethoxysilan (GENIOSIL XL 42), (Isocyanatomethyl)trimethoxysilan (GENIOSIL XL 43) 3-(Triethoxysilyl)propylbernsteinsäureanhydrid (GENIOSIL GF 20), (Methacryloxymethyl)methyldiethoxysilan, 2-Acryloxyethylmethyldimethoxysilan, 2-Methacryloxyethyltrimethoxysilan, 3-Acryloxypropylmethyldimethoxysilan, 2-Acryloxyethyltrimethoxysilan, 2-Methacryloxyethyltriethoxysilan, 3-Acryloxypropyltrimethoxysilan, 3-Acryloxypropyltripropoxysilan, 3-Methacryloxypropyltriethoxysilan, 3-Methacryloxypropyltriacetoxysilan, 3-Methacryloxypropymethyldimethoxysilan, Vinyltrichlorsilan, Vinyltrimethoxysilan (GENIOSIL XL 10), Vinyltris(2-methoxyethoxy)silan (GENIOSIL GF 58), Vinyltriacetoxysilan.

Es ist aber auch möglich, andere organofunktionelle Silane auf den erfindungsgemäßen Effektpigmenten zu verwenden.

Weiterhin lassen sich, beispielsweise kommerziell von Degussa erhältliche, wäßrige Vorhydrolysate einsetzen. Hierzu gehören u.a. wässriges, alkoholfreies Aminosilanhydrolysat (Dynasylan Hydrosil 1151), wässriges, alkoholfreies amino/alkylfunktionelles Siloxancooligomer (Dynasylan Hydrosil 2627), wässriges, alkoholfreies diamino/alkylfunktionelles Siloxancooligomer (Dynasylan Hydrosil 2776), wässriges, alkoholfreies amino/vinyifunktionelles Siloxancooligomer (Dynasylan Hydrosil 2907), wässriges, alkoholfreies amino/alkylfunktionelles Siloxancooligomer (Dynasylan Hydrosil 2909), wässriges, alkoholfreies epoxyfunktionelles Siloxanoligomer (Dynasylan Hydrosil 2926) oder wässriges, alkoholfreies amino/methacrylatfunktionelles Siloxancooligomer (Dynasylan Hydrosil 2929), oligomeres Diaminosilansystem (Dynasylan 1146), vinyl-/alkylfunktionelles Siloxancooligomer (Dynasylan 6598), vinyl- und methoxygruppenhaltiges Vinylsilankonzentrat (oligomeres Siloxan) (Dynasylan 6490) oder oligomeres kurzkettiges alkylfunktionelles Silan (Dynasylan 9896).

Bei einer bevorzugten Ausführungsform enthält das organofunktionelle Silangemisch neben wenigstens einem Silan ohne funktionelle Bindungsgruppe wenigstens ein aminofunktionelles Silan. Die Aminofunktion ist eine funktionelle Gruppe, die mit den meisten in Bindemitteln vorhandenen Gruppen eine oder mehrere chemische Wechselwirkungen eingehen kann. Dies kann eine kovalente Bindung, wie z.B. mit Isocyanat- oder Carboxylatfunktionen des Bindemittels, oder Wasserstoffbrückenbindungen wie mit OH- oder COOR-Funktionen oder auch ionische Wechselwirkungen beinhalten. Eine Aminofunktion ist daher für den Zweck der chemischen Anbindung des Effektpigmentes an verschiedenartige Bindemittel sehr gut geeignet.

Bevorzugt werden hierzu folgende Verbindungen genommen:

Aminopropyltrimethoxysilan (Dynasylan AMMO; Silquest A-1110), Aminopropyltriethoxysilan (Dynasylan AMEO) oder N-(2-Aminoethyl)-3-aminopropyltrimethoxysilan (Dynasylan DAMO, Silquest A-1120) oder N-(2-Aminoethyl)-3-aminopropyltriethoxysilan, Triamino-funktionelles Trimethoxysilan (Silquest A-1130), bis-(gamma-Trimethoxysilylpropyl)amin (Silquest A-1170), N-ethyl-gamma-aminoisobutyltrimethoxysilan (Silquest A-Link 15), N-Phenyl-gammaaminopropyltrimethoxysilan (Silquest Y-9669), 4-Amino-3,3-dimethylbutyltrimethoxysilan (Silquest Y-11637), N-Cyclohexylaminomethylmethyldiethoxysilan (GENIOSIL XL 924), (N-Cyclohexylaminomethyl)triethoxysilan (GENIOSIL XL 926), (N-Phenylaminomethyl)trimethoxysilan (GENIOSIL XL 973) und deren Mischungen.

Bei einer weiterhin bevorzugten Ausführungsform ist das Silan ohne funktionelle Bindungsgruppe ein Alkylsilan. Das Alkylsilan weist vorzugsweise die Formel (A) auf:

$$R_{(4-z)}Si(X)_z, \qquad (A)$$

Hierbei ist z eine ganze Zahl von 1 bis 3, R ist eine substituierte oder unsubstituierte, unverzweigte oder verzweigte Alkylkette mit 10 bis 22 C-Atomen und X steht für eine Halogen- und/oder Alkoxygruppe. Bevorzugt sind Alkylsilane mit Alkylketten mit mindestens 12 C-Atomen. R kann auch zyklisch mit Si verbunden sein, wobei in diesem Fall z üblicherweise 2 ist.

Ein derartiges Silan bewirkt eine starke Hydrophobierung der Pigmentoberfläche. Diese wiederum führt dazu, daß das derart beschichtete Perlglanzpigment in der Lackbeschichtung tendenziell nach oben aufschwimmt. Bei plättchenförmigen Effektpigmenten wird ein derartiges Verhalten als "leafing"-Verhalten bezeichnet.

Eine Silanmischung bestehend aus mindestens einem Silan, welches wenigstens eine funktionelle Gruppe besitzt, die eine Anbindung an das Bindemittel ermöglicht, und einem, in Wasser unlöslichen oder kaum löslichen Alkylsilan ohne Aminogruppe ermöglicht optimale anwendungstechnische Eigenschaften der Perlglanzpigmente.

Eine solche organisch-chemischen Oberflächenmodifizierung führt dazu, dass sich die Effektpigmente in einer Lack- oder Farbschicht ausgezeichnet orientieren, d.h. im Wesentlichen planparallel zu dem lackierten bzw. angestrichenen Untergrund, und zugleich chemisch mit dem Bindemittelsystem des Lackes bzw. der Farbe reagieren und mithin kovalent in der Lack- bzw. Farbschicht gebunden sind. Derartige Lack- bzw. Farbschichten weisen eine erhöhte mechanische und chemische Beständigkeit gegenüber Umwelteinflüssen, wie Wetter etc., auf.

Werden die erfindungsgemäßen Effektpigmente in einem Rakelabzug (z.B. im Nitrocelluloselack) unter einem Lichtmikroskop in der Durchsicht betrachtet, so erkennt man bei den erfindungsgemäßen Effektpigmenten, dass sie eine sehr einheitliche Farbe aufweisen. Die einzelnen Pigmente weisen weitgehend die gleiche Farbe, vorzugsweise die gleiche Farbe, auf.

Die Farbgebung von Effektpigmenten, wie Perlglanzpigmenten, erfolgt durch Interferenzphänomene. Einander komplementäre Interferenzfarben löschen sich dabei gegenseitig aus, so erzeugt beispielsweise eine Mischung von blauen und gelben Perlglanzpigmenten einen silbernen oder grauen Farbeindruck. Gut bekannt ist, dass Mischungen verschiedenfarbiger Perlglanzpigmente stets einen unreineren Farbton mit geringerem Glanz ergeben, da sich immer einige Farbanteile gegenseitig auslöschen bzw. schwächen.

Um Effektpigmente, vorzugsweise Perlglanzpigmente, mit hoher Farbreinheit zu erhalten, müssen mithin die Farbtöne der einzelnen Pigmente möglichst einheitlich sein. Die Farbtonreinheit kann sich durch folgendes Verfahren nach einer statistischen Auswertung quantitativ darstellen lassen.

Ausgangspunkt zur Bestimmung der Farbreinheit der erfindungsgemäßen Effektpigmente sind farbige digitale Mikroskopbilder von Rakelabzügen. Der Rakelabzug wird mit einem Lack, enthaltend 6 Gew.-%-Effektpigmente in einem farblosen Nitrocelluloselack, durchgeführt. Die Mengenangaben Gew.-% bezieht sich dabei auf das Gesamtgewicht des Lackes. Der Lack wird in einer Naßfilmdicke von 76 $\mu$m auf eine BYK-Gardner Schwarz-Weiß-Rakeikarte (byko-chart 2853) aufgebracht und nachfolgend getrocknet. Innerhalb dieser ausgetrockneten Rakelabzüge sind die plättchenförmigen Effektpigmente weitgehend planparallel orientiert. Aufgenommen werden die Mikroskopbilder bevorzugt mit dem Mikroskop Axioskop 2 und der Digitalkamera AxioCam der Fa. Zeiss, Deutschland. Von den erhaltenen Digitalbildern werden sodann mit Hilfe der Bildaufnahme- und Bildverarbeitungs-Software AxioVision 4.6 der Fa. Zeiss farbneutrale Digitalbilder mit 12 Bit Farbtiefe angefertigt. Bei der Betrachtung der Rakelabzüge durch das Mikroskop fällt das eingestrahlte Licht von oben senkrecht auf den Rakelabzug. In dem Rakelabzug wiederum sind die Effektpigmente weitgehend planparallel orientiert und mithin fällt das einfallende Licht senkrecht auf die Pigmentplättchen. Der Beobachtungswinkel ist in diesem Fall ebenfalls senkrecht zur Oberfläche der Pigmentplättchen.

Um eine verfälschungsfreie Statistik zu erhalten, wurden ca. 20 Einzelaufnahmen an zufällig ausgewählten Stellen auf dem schwarzen Untergrund der Rakelabzüge erstellt. Dabei wurden über 100 auswertbare Einzelpigmente abgebildet.

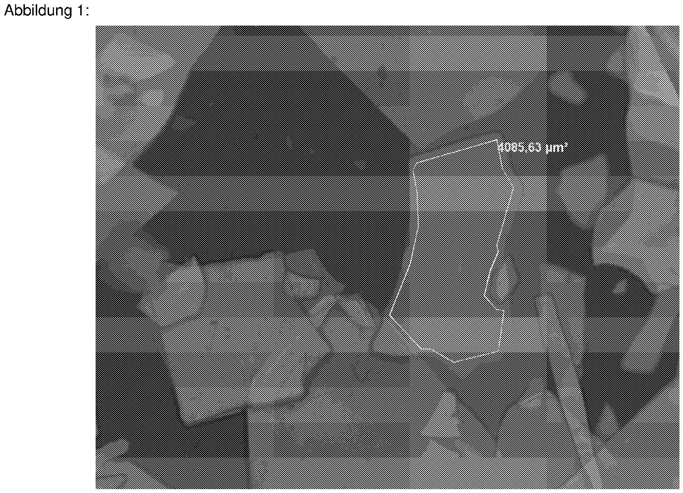

Da die Bildverarbeitungssoftware die Pigmente nicht automatisch erkennen kann, werden diese manuell markiert. Bei der Markierung der einzelnen Pigmentflächenanteile ist zu beachten, dass keine Pigmentrandbereiche bzw. Bereiche, in denen sich die Pigmente überlappen, genommen werden, da in diesem Fall der Farbeindruck der einzelnen Pigmentteilchen durch die Überlappung sofort verfälscht ist (s. auch Abbildung 1).

Bei den so markierten Flächen berechnet die Bildverarbeitungssoftware die jeweiligen Flächenmittelwerte der Rot-, Grün- und Blauanteile. Um eine aussagefähige Statistik zu werden die Farbanteile von mindestens 100 auswertbaren Pigmentteilchen aus den verschiedenen Mikroskopaufnahmen bestimmt.

In der Bildverarbeitungssoftware wird das sRGB-Farbmodell verwendet. Die Umrechnung in den XYZ-Farbraum erfolgt über die allgemein bekannte Transformationsmatrix (II) (IEC 61966-2-1 :1999).

$$\begin{pmatrix} X \\ Y \\ Z \end{pmatrix} = \begin{pmatrix} 0{,}4124 & 0{,}3576 & 0{,}1805 \\ 0{,}2126 & 0{,}7152 & 0{,}0722 \\ 0{,}0193 & 0{,}1192 & 0{,}9505 \end{pmatrix} \begin{pmatrix} R \\ G \\ B \end{pmatrix} \qquad \text{(II)}$$

Die so erhaltenen X, Y und Z-Werte werden anschließend in die gebräuchlichen CIE (1976) Lab-Werte umgerechnet:

$$L^* = 116\, Y^* - 16 \qquad\qquad (III)$$

$$a^* = 500\,(\, X^* - Y^*\,) \qquad\qquad (IV)$$

$$b^* = 200\,(\, Y^* - Z^*\,) \qquad\qquad (V)$$

$$X^* = \sqrt[3]{\dfrac{X}{X_n}} \qquad\qquad (VI)$$

Die Berechnung für $Y^*$ und $Z^*$ erfolgt analog der Berechnung von $X^*$. Es wurden die Normfarbwerte $X_n = 94{,}81$, $Y_n = 100$ und $Z_n = 107{,}34$ für die Lichtart D65 und den 10°-Normalbeobachter verwendet.

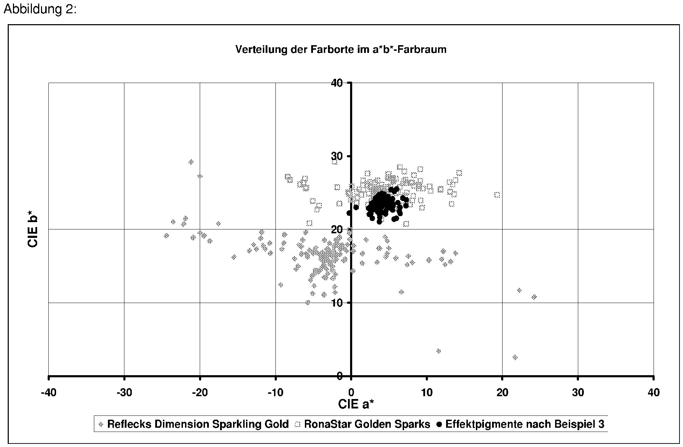

Man erhält so eine Verteilung von Meßpunkten im a\*-b\*-Farbraum (s. auch die Abbildung 2): Durch den Abstand der Meßpunkte vom Ursprung des a\*,b\*-Diagramms ist die Farbsättigung (Chroma) $C^*$ jedes einzelnen Meßwertes festgelegt.

Zur Quantifizierung der Streuung wird zunächst der gemeinsame Mittelpunkt $(\overline{a},\overline{b})$ der Messwerte im a\*-b\*-Farbraum nach den Formeln (VIIa; VIIb) berechnet:

$$\overline{a} = \sum_i a_i \qquad (VIIa)$$

$$\overline{b} = \sum_i b_i \qquad (VIIb)$$

Anschließend wird für jeden Meßpunkt (a,b) dessen Abstand zu dem Mittelpunkt $(\overline{a},\overline{b})$ als Absolutwert $\Delta C^*$ in Form der Wurzel des Quadrates der Differenz der Werte ermittelt (VIII):

$$\Delta C^* = \sqrt{(a - \overline{a})^2 + (b - \overline{b})^2} \qquad\qquad (VIII)$$

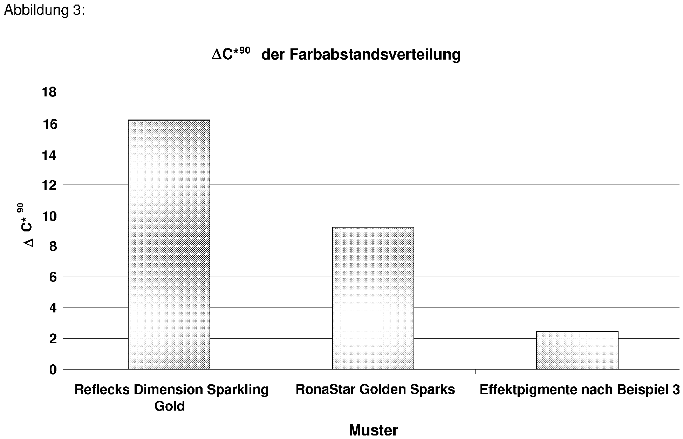

Man erhält eine Farbabstandsverteilung im a\*-b\*-Farbraum. Diese lässt sich ebenfalls als Summendurchgangsverteilungskurve darstellen. Das 90%-Quantil ($\Delta C^*_{90}$) dieser Farbabstandsverteilung ist ein geeignetes Maß, um die Größe der Farbstreuung zu charakterisieren (s. Abbildung 3). Dieser Wert markiert jenen Farbabstand $\Delta C^*$ vom Mittelwert unterhalb dessen 90 % der gezählten Pigmentteilchen liegen.

Die erfindungsgemäßen Effektpigmente weisen vorzugsweise ein 90%-Quantil der Farbabstandsverteilung $\Delta C^*_{90}$ von 0,2 bis 8,0 und bevorzugt von 0,5 bis 6,0; weiter bevorzugt von 0,6 bis 5,0; besonders bevorzugt von 0,7 bis 4,0 und ganz besonders bevorzugt von 0,7 bis 3,0 auf.

Die Erfinder haben für diesen überraschenden Effekt noch keine wissenschaftlich aussagefähige Erklärung gefunden. Es wird jedoch vermutet, dass die in ihrer Größe weitgehend gleichen Pigmentsubstrate wesentlich gleichmäßiger mit den farbgebenden Schichten wie beispielsweise hochbrechende Metalloxide oder semitransparente Metalle beschichtet werden, als dies bei Substraten mit höherem Span $\Delta D$ der Fall ist. Dadurch bilden sich auf den Substraten Beschichtungen mit einer wesentlich homogeneren Schichtdicke aus. Da die Farbgebung durch Interferenzphänomene erzeugt und diese bei den verwendeten Substraten entscheidend von der Schichtdicke der hochbrechenden Beschichtung bestimmt wird, ergeben die gleichmäßigeren Schichtdicken einen reineren Farbton bzw. eine größere Farbreinheit bei den erfindungsgemäßen Effektpigmenten.

Die vorliegende Erfindung umfasst Perlglanzpigmente ohne Farbflop sowie auch Effektpigmente mit einem hohen Farbflop bei Multilayersystemen.

Entscheidend ist jedoch, dass bei konstantem Einfallswinkel und Beobachtungswinkel in einem Rakelabzug bei weitgehend planparalleler Orientierung der erfindungsgemäßen plättchenförmigen Effektpigmente stets ein weitgehend gleicher Farbton im Sinne der vorstehenden Beschreibung beobachtet wird. Dabei muß der Einfalls- bzw. Beobachtungswinkel keineswegs immer senkrecht zu den Pigmentplättchen sein, wenngleich dieser Fall auch bevorzugt ist.

Die erfindungsgemäßen Effektpigmente eignen sich insbesondere für die Anwendung in Kosmetika, wie z.B. Körperpuder, Gesichtspuder, gepresstem und losem Puder, Gesichtsmakeup, Pudercreme, Crememakeup, Emulsionsmakeup, Wachsmakeup, Foundation, Moussemakeup, Wangenrouge, Augenmakeup wie Lidschatten, Mascara, Eyeliner, flüssige Eyeliner, Augenbrauenstift, Lippenpflegestift, Lippenstift, Lip Gloss, Lip Liner, Haarstylingkompositionen wie Haarspray, Haarmousse, Haargel, Haarwachs, Haarmascara, permanente oder semi-permanente Haarfarben, temporäre Haarfarben, Hautpflegekompositionen wie Lotions, Gele, Emulsionen sowie Nagellackkompositionen.

Zur Erzielung spezieller Farbeffekte können in den Kosmetikapplikationen neben den erfindungsgemäßen Effektpigmenten weitere Farbmittel und/oder herkömmliche Effektpigmente oder Mischungen hiervon in variablen Mengenverhältnissen eingesetzt werden. Als herkömmliche Effektpigmente können beispielsweise handelsübliche Perlglanzpigmente auf der Basis von mit hochbrechenden Metalloxiden beschichtetem natürlichen Glimmer (wie z.B. die Produktgruppe Prestige® der Fa. Eckart), BiOCl-Plättchen, TiO$_2$-Plättchen, Perlglanzpigmente auf der Basis von mit hochbrechenden Metalloxiden beschichtetem synthetischen Glimmer oder basierend auf mit hochbrechenden Metalloxiden beschichteten Glasplättchen, Al$_2$O$_3$-, SiO$_2$- oder TiO$_2$-Plättchen verwendet werden. Außerdem können auch Metalleffektpigmente, wie z.B. die Produktgruppe Visionaire® der Fa. Eckart, zugegeben werden. Die Farbmittel können aus anorganischen oder organischen Pigmenten ausgewählt sein.

Weiter bevorzugte Effektpigmente im Sinne der vorliegenden Erfindung besitzen einen Span ΔD von 0,8 - 1,2, einen $D_{50}$-Wert von jeweils vorzugsweise 10 - 35 μm, 30 - 65 μm, 40 - 140 μm oder 135 - 250 μm. Ihre mittlere Dicke beträgt dabei 500 -2000 nm, und die Standardabweichung in der Dicke beträgt dabei 15 - 100%.

Weiter bevorzugte Effektpigmente im Sinne der vorliegenden Erfindung besitzen einen Span ΔD von 0,8 - 1,2 sowie eine Standardabweichung in der Dicke von 20 - 70 %. Die mittlere Dicke der Effektpigmente beträgt dabei 500 - 2000 nm, bevorzugt 750 - 1500 nm.

Ein Verfahren zur Herstellung der erfindungsgemäßen Effektpigmente umfasst die folgenden Schritte:

a) Größenklassieren der künstlichen Substrate
b) Beschichten der künstlichen Substrate.

Wenn die Ausgangssubstrate zu groß sind, kann optional ein Zerkleinerungsschritt vor dem Größenklassieren durchgeführt werden.

Die Größenklassierung kann vor oder nach der Beschichtung der Substrate erfolgen. Vorteilhafterweise wird jedoch zuerst das Substrat klassiert und anschließend beschichtet. Die Größenklassierung wird durchgeführt und gegebenenfalls wiederholt, bis die Perlglanzpigmente die erfindungsgemäße Teilchengrößenverteilung aufweisen.

Ein enger Span ΔD der Substrate kann durch geeignete Zerkleinerungs- und/oder Klassierungsprozesse der zu beschichtenden, künstlichen Substrate erreicht werden. Die zu beschichtenden künstlichen Substrate können beispielsweise durch Kugelmühle, Strahl- oder Rührwerkskugelmühle, Kollergang oder Dissolver zerkleinert werden. Der Span ΔD der Endfraktion kann durch geeignete Klassierung, wie beispielsweise eine mehrfache Naßsiebung, eingestellt werden. Weitere Klassierungsverfahren umfassen die Zentrifugation in Zyklonen oder eine Sedimentation aus einer Dispersion.

Die Zerkleinerungs- und Klassierprozesse können nacheinander erfolgen und gegebenenfalls miteinander kombiniert werden. So kann auf einen Zerkleinerungsvorgang ein Klassierungsvorgang folgen, dem sich ein weiterer Zerkleinerungsvorgang des Feinguts anschließt usw..

Die Effektpigmente nach einem der Ansprüche 1 bis 14 können in einer Beschichtungszusammensetzung verwendet werden. Gemäß einer bevorzugten Variante wird die Beschichtungszusammensetzung aus der Gruppe, die aus Kosmetikum, Farbe, Druckfarbe, Lack, Pulverlack und Elektrotauchlack besteht, ausgewählt.

Die Effektpigmente nach einem der Ansprüche 1 bis 14 können in einem Kunststoff verwendet werden.

Im Folgenden wird die Erfindung durch einige Beispiele und Abbildungen näher erläutert, ohne auf diese beschränkt zu sein.

**Abbildungen**

Abb. 1 zeigt einen manuell markierten Flächenanteil in einem Digitalbild eines erfindungsgemäßen Effektpigmentes in einem getrockneten Rakelauszug.

Abb. 2 zeigt die Verteilung der aus den Digitalbildern der manuell markierten Effektpigmente berechneten Farborte in einem a*b*-Farbraum.

Abb. 3 zeigt das 90 % Quantil $\Delta C^*_{90}$ der Farbabstandsverteilung

Die folgenden Beispiele sollen die Erfindung näher erläutern, ohne sie jedoch auf diese einzuschränken.

**Beispiel 1:**

Eine Suspension von 200 g Glasplättchen (mittlere Dicke: 1 $\mu$m, Standardabweichung der Dicke: ca. 40%) in VE-Wasser (ca. 3 Gew.-%ig) (VE: vollentsalzt) wird über ein 100 $\mu$m Sieb klassiert und der Siebdurchgang wiederum über ein 63 $\mu$m Sieb gesiebt. Diese Siebprozedur wird mit auf dem 63 $\mu$m Sieb erhaltenen Siebrückstand zweimalig wiederholt und so wird eine Glasplättchenfraktion erhalten, die folgende Partikelgrößenverteilung aufweist (MALVERN Mastersizer MS 2000): $D_{10}$=50 $\mu$m, $D_{50}$=82 $\mu$m, $D_{90}$= 132 $\mu$m, Span $\Delta D$ = 1,00.

**Beispiel 2:**

200 g Glasplättchen aus Beispiel 1 werden in 2000 ml VE-Wasser suspendiert und unter turbulentem Rühren auf 80 °C erhitzt. Der pH-Wert der Suspension wird mit verdünnter HCl auf 1,9 eingestellt und sodann eine erste Schicht "$SnO_2$" auf den Glasplättchen gefällt. Diese Schicht wird durch Zugabe von einer Lösung, bestehend aus 3 g $SnCl_4$ x 5 $H_2O$ (in 10 ml konz. HCl plus 50 ml VE-Wasser), unter gleichzeitiger Zudosierung von einer 10% NaOH, um den pH-Wert konstant zu halten, über einen Zeitraum von 1 h gebildet. Um die Fällung zu vervollständigen wird die Suspension noch weitere 15 min gerührt. Danach wird der pH-Wert mit verdünnter HCl auf pH 1,6 heruntergesetzt und sodann eine Lösung von 200 ml $TiCl_4$ (400 g$TiCl_4$/l VE-Wasser) in die Suspension hinzudosiert. Der pH-Wert wird dabei durch Gegensteuern mit 10% NaOH konstant auf pH 1,6 gehalten. Danach wird noch 15 min nachgerührt, abfiltriert und der Filterkuchen mit VE-Wasser nachgewaschen. Der Filterkuchen wird dann bei 100 °C vorgetrocknet und bei 750 °C 30 min kalziniert. Es wird ein hochglänzendes Effektpigment erhalten, welches eine rote Interferenzfarbe zeigt. Eine mikroskopische Untersuchung der beschichteten Pigmente zeigt eine außergewöhnliche Farbkonstanz der Pigmente untereinander.

**Beispiel 3:**

200 g Glasplättchen aus Beispiel 1 werden analog Beispiel 2 mit $TiO_2$ beschichtet, allerdings werden nur 167 ml TiCl4 zudosiert. Man erhält nach Kalzination ein hochglänzendes Effektpigment mit einer goldenen Interferenzfarbe. Eine mikroskopische Untersuchung zeigt analog zu Beispiel 2 eine außergewöhnliche Farbkonstanz der Pigmente untereinander.

**Vergleichsbeispiel 4:**

Kommerziell erhältliche goldene Perlglanzpigmente "Reflecks Dimension Sparkling Gold" der Fa. BASF Catalysts.

**Vergleichsbeispiel 5:**

Kommerziell erhältliche goldene Perlglanzpigmente "RonaStar Golden Sparks" der Fa. Merck.

Tab. 3: Kennwerte der Größenverteilung für einige Beispiele und Vergleichsbeispiele:

| Pigment | $D_{10}$ | $D_{50}$ | $D_{90}$ | Span $\Delta D$ |
|---|---|---|---|---|
| Ronastar Golden Sparks (Vergleichsbeispiel 4) | 34,0 | 78,4 | 148,7 | 1,46 |
| Reflecks Dimension Sparkling Gold (Vergleichsbeispiel 5) | 35,6 | 84,3 | 175,9 | 1,66 |
| Beispiel 3 | 49,8 | 81,5 | 130,7 | 0,99 |

Im Folgenden wurden die erfindungsgemäßen Effektpigmente des Beispiels 3 mit engem Span und hoher Farbreinheit mit den kommerziell erhältlichen Perlglanzpigmenten der Vergleichsbeispiele in einer detaillierten Analyse verglichen. Alle Perlglanzpigmente weisen im Rakelabzug eine für den Beobachter visuell wahrnehmbare goldene Interferenzfarbe auf,

Ausgangspunkt zur Bestimmung der Farbreinheit der erfindungsgemäßen Effektpigmente waren farbige digitale Mikroskopbilder von getrockneten Rakelabzügen (hergestellt mit einem Lack, der 6 Gew.-%-Pigmente in Nitrocelluloselack enthält (Gew.-% Angabe bezieht sich auf das Gesamtgewicht des Lackes) und in einer Naßfilmdicke von 76 $\mu$m

auf BYK-Gardner Schwarz-Weiß-Rakelkarte (byko-chart 2853) aufgerakelt und nachfolgend bei Raumtemperatur getrocknet wurden). Aufgenommen wurden diese mit dem Mikroskop Axioskop 2 und der Digitalkamera AxioCam der Fa. Zeiss. Diese Ausstattung erlaubte es, mit Hilfe der Bildaufnahme- und Bildverarbeitungs-Software AxioVision 4.6 farbneutrale Digitalbilder mit 12 Bit Farbtiefe anzufertigen.

Um eine verfälschungsfreie Statistik zu erhalten, wurden ca. 20 Einzelaufnahmen an zufällig ausgewählten Stellen der Rakelabzüge erstellt. Dabei wurden über 100 auswertbare Einzelpigmente abgebildet.

In Abb. 2 sind die Farborte der Einzelmessungen der drei Proben im a*,b*-Diagramm aufgetragen. Deutlich zu erkennen ist, dass die erfindungsgemäßen Effektpigmente im Unterschied zu den kommerziell erhältlichen Produkten die geringste Streuung im a*-b*-Farbraum aufweisen, also am farbreinsten sind. Die Farbwerte liegen dicht beisammen im rot-gelben Quadranten des a*-b*-Farbraums. Die größte Streuung, also die am stärksten uneinheitlich gefärbten Pigmente sind bei den Pigmenten des Vergleichsbeispiels 4 zu beobachten. Dies ist auch bereits mit bloßem Auge an den Rakelabzügen zu erkennen. Eine etwas engere Streuung, aber dennoch eine Verteilung über den grün- und rotgelben Quadranten des a*-b*-Farbraums ist bei den Merck Pigmenten Ronastar (Fa. Merck, Darmstadt, Deutschland) vorhanden.

Tabelle 4: $\Delta C^*_{90}$-Quantil

| Pigment | $\Delta C^*_{90}$-Quantil |
|---|---|
| Ronastar Golden Sparks (Vergleichsbeispiel 4) | 9,2 |
| Reflecks Dimension Sparkling Gold (Vergleichsbeispiel 5) | 16,2 |
| Beispiel 3 | 2,5 |

Das 90%-Quantil der Farbabstandsverteilung ist für die erfindungsgemäßen Effektpigmente des erfindungsgemäßen Beispiels 3 am geringsten, während die kommerziell erhältlichen Vergleichsprodukte deutlich größere Werte aufweisen.

**Beispiel 6:**

200 g Glasplättchen aus Beispiel 1 werden in 2000 ml VE-Wasser suspendiert und unter turbulentem Rühren auf 80 °C erhitzt. Der pH-Wert der Suspension wird mit verdünnter HCl auf pH 1,9 eingestellt und sodann eine erste Schicht "$SnO_2$" auf den Glasplättchen gefällt. Diese Schicht wird durch Zugabe von einer Lösung, bestehend aus 3 g $SnCl_4$ x 5 $H_2O$ (in 10 ml konz. HCl plus 50 ml VE-Wasser), unter gleichzeitiger Zudosierung von einer 10% NaOH, um den pH-Wert konstant zu halten, über den Zeitraum von 1 h gebildet. Um die Fällung zu vervollständigen wird die Suspension noch weitere 15 min gerührt. Danach wird der pH-Wert mit verdünnter HCl auf pH 1,6 heruntergesetzt und sodann eine Lösung von 185 ml $TiCl_4$ (400 g$TiCl_4$/l VE-Wasser) in die Suspension hinzudosiert. Der pH-Wert wird dabei durch Gegensteuern mit 10% NaOH konstant auf pH 1,6 gehalten. Danach wird der pH-Wert mit 5 % NaOH auf 7,5 angehoben und 15 min gerührt. Eine Wasserglaslösung (207 g Wasserglaslösung, 27% $SiO_2$, gemischt mit 207 g VE-Wasser) wird sodann langsam in die Suspension eingeleitet und der pH-Wert konstant bei pH 7,5 gehalten. Danach wird noch 20 min nachgerührt und der pH-Wert wieder auf 1,9 gesenkt. Dann scheidet man eine zweite Schicht "$SnO_2$" auf der $SiO_2$-Oberfläche ab. Diese Schicht wird durch Zugabe von einer Lösung, bestehend aus 3 g $SnCl_4$ x 5 $H_2O$ (in 10 ml konz. HCl plus 50 ml VE-Wasser), unter gleichzeitiger Zudosierung von einer 10% NaOH, um den pH-Wert konstant zu halten, über den Zeitraum von 1 h gebildet. Um die Fällung zu vervollständigen wird die Suspension noch weitere 15 min gerührt. Danach wird der pH-Wert mit verdünnter HCl auf pH 1,6 heruntergesetzt und sodann eine Lösung von 189 ml $TiCl_4$ (400 g$TiCl_4$/l VE-Wasser) in die Suspension hinzudosiert. Der pH-Wert wird dabei durch Gegensteuern mit 10 % NaOH konstant auf pH 1,6 gehalten. Danach wird noch 15 min nachgerührt, abfiltriert und der Filterkuchen mit VE-Wasser nachgewaschen. Der Filterkuchen wird bei 100 °C vorgetrocknet und bei 750 °C 30 min kalziniert. Es wird ein extrem hochglänzendes Effektpigment erhalten, welches einen Farbflop aufweist, der von einer grünen Interferenzfarbe für steile Betrachtungswinkel in eine blaue Interferenzfarbe bei flachem Betrachtungswinkel wechselt.

Im Folgenden werden kosmetische Applikationen mit den erfindungsgemäßen Effektpigmenten vorgestellt:

**Beispiel 7: Body Powder**

| INCI Name | Product Name | %W/W | Supplier |
|---|---|---|---|
| A | | 100.00 | |
| Mica | Silk Mica | ad 100 | www.vwr.com |

(fortgesetzt)

| INCI Name | Product Name | %W/W | Supplier |
|---|---|---|---|
| Talc | Talc Powder | 18.00 | www.riedeldehaen.com |
| Boron Nitride | Softouch CCS 102 | 5.00 | www.advceramics.com |
| Nylon 12 | Orgasol 2002 D/Nat | 8.00 | www.atofinachemicals.com |
| Magnesium Stearate | Magnesium Stearate | 6.00 | www.sigmaaldrich.com |
| Methylparaben, Propylparaben | Rokonsal SSH-1 | 0.30 | www.biochema.com |
| Mica (and) Iron Oxides | Prestige® Soft Bronze | 9.00 | www.eckart.net |
| **Effektpigment nach Beispiel 2/3** | **--** | **0.10-15.00** | |
| Mica (and) Titanium Dioxide | Prestige® Magic Orange | 9.00 | www.eckart.net |
| B | | | |
| Tridecyl Stearate (and) Tridecyl Trimellitate (and) Dipentaerythrityl Hexacaprylate/Hexacaprate | Lipovol MOS-130 | 2.00 | www.lipochemicals.com |

Zubereitung:

1. Komponenten von Phase A mischen

2. Phase B zu Phase A hinzugeben

3. Mischen und in geeignetes Gefäß abfüllen

### Beispiel 8: Cream Eye Shadow

| INCI Name | Product Name | %W/W | Supplier |
|---|---|---|---|
| A | | 100.00 | |
| Castor Oil | Castor Oil | ad 100 | www.riedeldehaen.com |
| Octyl Palmitate | Liponate EHP | 6.00 | www.lipochemicals.com |
| Cocos Nucifera (Coconut) Oil | Lipovol C-76 | 7.00 | www.lipochemicals.com |
| Bees Wax | Ewacera 12 | 6.00 | www.wagnerlanolin.com |
| Isopropyl Lanolate | Ewalan IP | 5.00 | www.wagnerlanolin.com |
| Persea Gratissima (Avocado) Oil and Hydrogenated Avocado Oil | Avocado Butter | 7.00 | www.impag.de |
| Magnesium Stearate | Magnesium Stearate | 3.00 | www.sigmaaldrich.com |
| Bis-Hydroxyethoxypropyl Dimethicone | Dow Corning 5562 Carbinol Fluid | 7.00 | www.dowcorning.com |
| Dimethicone/ Vinyl Dimethicone Crosspolymer and Silica | Dow Corning 9701 Cosmetic Powder | 5.00 | www.dowcorning .com |
| Phenoxyethanol (and) Methylparaben (and) Ethylparaben (and) Butylparaben | Uniphen P-23 | 0.30 | www.induchem.com |

(fortgesetzt)

| INCI Name | Product Name | %W/W | Supplier |
|---|---|---|---|
| B | | | |
| Mica (and) Iron Oxides | Prestige® Soft Bronze | 21.00 | www.eckart.net |
| **Effektpigment nach Beispiel 2/ 3** | ----- | **0.10 - 20.00** | |

Zubereitung:

1. Phase A mischen und auf 85°C erwärmen

2. Phase B mischen

3. Unter Rühren Phase B zu Phase A hinzugeben

4. In geeignetem Gefäß auf Raumtemperatur abkühlen lassen

### Beispiel 9: Foundation

| INCI Name | Product Name | %W/W | Supplier |
|---|---|---|---|
| A | | 100.00 | |
| Hydrogenated Polydecene | Ritadecene 20 | 9.00 | www.ritacorp.com |
| Caprylic/Capric Triglyceride | Liponate GC-K | 5.00 | www.lipochemicals.com |
| Prunus Amygdalus Dulcis (Sweet Almond) Oil | SweetAlmond Oil | 4.00 | www.jandekker.com |
| Caprylyl Trimethicone | SilCare Silicone 31M50 | 4.00 | www.clariant.com |
| Caprylyl Methicone | SilCare Silicone 41M15 | 3.00 | www.clariant.com |
| Steareth-2 | Volpo S2 | 1.60 | www.croda.com |
| Steareth-20 | Sympatens AS/200 G | 2,40 | www.kolb.ch |
| B | | | |
| Talc | Talc Powder | 4.50 | www.vwr.com |
| Mica (and) Iron Oxides | Prestige®Soft Beige | 4.00 | www.eckart.net |
| Mica (and) Titanium Dioxide | Prestige®Soft Silver | 1.00 | www.eckart.net |
| **Effektpigment nach Beispiel 2/ 3** | ------------------ | **0.05 - 1.00** | |
| C | | | |
| Glycerin | Pricerine 9090 | 5.00 | www.brenntag. com |

(fortgesetzt)

| INCI Name | Product Name | %W/W | Supplier |
|---|---|---|---|
| Water | Aqua | ad 100 | |
| Ammonium Acryloyldimehtyltaurate/ VP Copolymer | Aristoflex AVC | 0.40 | www.simon-und-werner.com |
| D | | | |
| Propylene Glycol (and) Diazolidinyl Urea (and) Methylparaben (and) Propylparaben | Nipaguard PDU | 0.40 | www.simon-und-werner.com |

Zubereitung:

1. Phase A unter Rühren auf 70°C erwämen

2. Komponenten der Phase B zu Phase A hinzufügen

3. Phase C mischen bis Aristoflex gelöst ist

4. Die Mischung auf 70°C erwärmen

5. Phase C zu Phase AB geben,

6. Auf 40°C abkühlen lassen und Phase D hinzufügen

**Beispiel 10: Lip Gloss**

| INCI Name | Product Name | %W/W | Supplier |
|---|---|---|---|
| A | | 100.00 | |
| Hydrogenated Polyisobutene (and) Ethylene/Propylene/ Styrene Copolymer (and) Butylene/Ethylene/Styrene Copolymer | Versagel ME 750 | ad 100 | www.penreco.com |
| Simmondsia Chinensis (Jojoba) Seed Oil | Jojoba Oil - Natural/Golden | 2.00 | www.biochemica.com |
| Caprylyl Trimethicone | Silcare Silicone 31M50 | 7.00 | www.clariant.com |
| Stearyl Dimethicone | Silcare Silicone 41M65 | 3.20 | www.clariant.com |
| Hydrogenated Polydecene | Nexbase 2002 | 4.00 | www.jandekker.com |
| Isopropyl Myristate | Isopropyl Myristate | 4.50 | www.vwr.com |
| B | | | |
| **Effektpigment nach Beispiel 2/ 3** | ----------------- | **0.01 - 2.00** | |
| Propylparaben | Propyl-4-hydroxybenzoat | 0.20 | www.sigmaaldrich.com |

Zubereitung:

1. Phase A auf 85°C erwärmen

2. Phase B zu Phase A hinzugeben, nach Vermischen in geeigentes Gefäß abfüllen

**Beispiel 11: Lip Liner**

| INCI Name | Product Name | %W/W | Supplier |
|---|---|---|---|
| A | | 100.00 | |
| Hydrogenated Coco-Glycerides | Softisan 100 | 12.35 | www.sasolwax.com |
| Candelilla Wax | Ewacera 42 | 14.00 | www.wagnerlanolin.de |
| Magnesium Stearate | Magnesium Stearate | 6.00 | www.sigmaaldrich.com |
| Stearic Acid | Kortacid 1895 | 8.50 | www.akzonobel.com |
| Hydrogenated Coconut Oil | Lipex 401 | 8.00 | www.kartshamns.com |
| Cetyl Palmitate | Walrath synthetic | 7.00 | www.kahlwax.de |
| Caprylic/Capric Triglyceride | Liponate GC-K | 3.60 | www.lipochemicals.com |
| Soybean Glycerides (and) Butyrospermum Parkii | Lipex L'sens | ad 100 | www.karlshamns.com |
| Tocopheryl Acetate | D,L-Alpha-Tocopherolacetat | 0.25 | www.dsm.com |
| Methylparaben; Propylparaben | Rokonsal SSH-1 | 0.30 | www.biochema.com |
| B | | | |
| Mica (and) Titanium Dioxide (and) Ferric Ferrocyanide | Prestige® Sapphire | 7.50 | www.eckart.net |
| Mica (and) Iron Oxides | Prestige® Copper | 7.50 | www.eckart.net |
| Mica (and) Titanium Dioxide | Prestige® Soft Silver | 5.00 | www.eckart.net |
| **Effektpigment nach Beispiel 2/ 3** | ----------------- | **0.10-10.00** | |

Zubereitung:

1. Phase A auf 85°C erwärmen

2. Phase B zu Phase A hinzugeben, nach Vermischen in geeignetes Gefäß abfüllen

**Beispiel 12: Lip-stick**

| INCI Name | Product Name | %W/W | Supplier |
|---|---|---|---|
| A | | 100.00 | |
| Camauba Wax | Ewacera 34 | 4.50 | www.wagnerlanolin.de |
| Bees Wax | Ewacera 12 | 3.50 | www.wagnerlanolin.de |
| Candelilla Wax | Ewacera 42 | 4.00 | www.wagnerlanolin.de |
| Microcrystalline Wax | Parcera MW | 7.20 | www.paramelt.com |

(fortgesetzt)

| INCI Name | Product Name | %W/W | Supplier |
|---|---|---|---|
| Cetyl Palmitate | Walrath synthetic | 2.00 | www.kahlwax.de |
| Hydrogenated Coco-Glycerides | Softisan 100 | 5.00 | www.sasolwax.com |
| Petrolatum | Penreco Blond | 5.80 | www.penreco.com |
| Cetearyl Octanoate | Luvitol EHO | 10.70 | www.basf.com |
| Tocopheryl Acetate | D,L-Alpha-Tocopherolacetat | 0.50 | www.dsm.com |
| Castor Oil | Castor Oil | ad 100 | www.riedetdehaen.com |
| B | | | |
| Mica (and) Iron Oxide | Prestige® Fire-red | 16.00 | www.eckart.net |
| **Effektpigment nach Beispiel 2/3** | ----------------- | **0.01 - 5.00** | |
| Methylparaben, Propylparaben | Rokonsal SSH-1 | 0.20 | www.biochema.com |

Zubereitung:

1. Phase A auf 85°C erwärmen

2. Phase B zu Phase A hinzugeben, nach Vermischen bei 75°C in geeignetes Gefäß abfüllen

**Beispiel 13: Liquid Eyeliner**

| INCI Name | Product Name | %W/W | Supplier |
|---|---|---|---|
| A | | 100.00 | |
| Water | Aqua | ad 100 | |
| Water/ carbon black dispersion | MBD 201 | 3.00 | www.geotech.nl |
| Acrylates Copolymer | Covacryl E14 | 10.00 | www.lcw.fr |
| Magnesium Aluminium Silicate | Veegum HV | 1.00 | www.cherbsloeh.de |
| B | | | |
| Propylene Glycol | 1,2 Propandiol | 3.00 | www.vwr.com |
| Triethanolamine | Triethanolamine | 1.40 | www.vwr.com |
| C | | | |
| Xanthan Gum | Keltrol T | 0.30 | www.cpkelco.com |
| D | | | |
| **Effektpigment nach Beispiel 2/3** | ----------------- | **0.10 - 5.00** | |
| Mica | Silk Mica | 2.00 | www.vwr.com |
| E | | | |
| Stearic Acid | Kortacid 1895 | 2.80 | www.akzonobef.de |
| Glyceryl Stearate | Aldo MS K FG | 0.80 | www.lonza.com |
| Oleyl Alcohol | HD-Ocenol 90/95 V | 0.50 | www.biesterfield.com |
| Phenoxyethanol (and) Methylparaben (and) Ethylparaben (and) Butylparaben | Uniphen P-23 | 0.50 | www.induchem.com |

(fortgesetzt)

| INCI Name | Product Name | %W/W | Supplier |
|---|---|---|---|
| F | | | |
| Dimethicone (and) Trisiloxane | Dow Corning 2-1184 Fluid | 5.00 | www.dowchemicals.com |

Zubereitung:

1. Veegum in Phase A dispergieren

2. 15 min rühren

3. Phase B zu Phase A hinzufügen

4. zu Phase AB Phase C geben

5. 10 Minuten rühren

6. Phase D zu Phase ABC geben und auf 75°C erwärmen

7. Phase E auf 75°C erwärmen

8. Phase E zu Phase ABCD geben

9. auf 60°C abkühlen lassen und Phase F hinzufügen

**Beispiel 14: Mascara**

| INCI Name | Product Name | %W/W | Supplier |
|---|---|---|---|
| A | | 100.00 | |
| Water | Aqua | ad 100 | |
| Propylene Glycol | 1, 2 Propanediol | 1.50 | www.vwr.com |
| **Effektpigment nach Beispiel 2/ 3** | ------------------ | **0.10 - 8.00** | |
| Cosmetic Black Oxide | C 33-134 Cosmetic Black Oxide | 7.00 | www.sunchemical.com |
| Caprylicl/Capric Triglyceride | Liponate GC-K | 2.00 | www.lipochemicals.com |
| Hydroxyethylcelfulose | Cellosize HEC QP-52000H | 0.30 | www.dow.com |
| Magnesium Alumium Silicate | Veegum HV | 1.80 | www.rtvanderbilt.com |
| B | | | |
| Carnauba Wax | Ewacera 34 | 3.00 | www.wagnerlanolin.de |
| Bees Wax | Ewacera 12 | 3.00 | www.wagnerlanolin.de |
| Glyceryl Stearate | Imwitor 960 K | 3.00 | www.sasolwax.xom |
| Cetyl Alcohol | Cetyl Alcohol | 3.80 | www.vwr.com |
| Polysorbate 60 | Tween 60 V | 1.00 | www.uniqema.com |
| PVP/ VA Copolymer | Luviskol VA 64 | 2.00 | www.basf.com |
| C | | | |

(fortgesetzt)

| INCI Name | Product Name | %W/W | Supplier |
|---|---|---|---|
| Water | Aqua | 4.00 | |
| BHT | Tenox BHT Kosher | 0.10 | www.eastman.com |
| Methylparaben | Methyl-4-hydroxybenzoate | 0.20 | www.sigmaaldrich.com |

Zubereitung:

1. Phase A unter Rühren auf 85°C erwärmen

2. Phase B auf 85°C erwärmen

3. Phase B zu Phase A hinzugeben

4. Unter Rühren auf 55°C abkühlen lassen

5. Phase C hinzufügen, vermischen, in geeignetes Gefäß abfüllen

**Beispiel 15: Mousse**

| INCI Name | Product Name | %W/W | Designated use |
|---|---|---|---|
| A | | 100.00 | |
| Cyclopentasiloxane | Dow Corning 245 Fluid | 8.60 | www.dowcorning.com |
| Hydrogenated Polyisobutene | MC 30 | 4.00 | www.sophim.com |
| Dimethicone (and) Dimethicone Crosspolymer | Dow Corning 9041 Silicone Elastomer Blend | ad 100 | www.dowcorning.com |
| Squalane | Squalane | 5.74 | www.impag.de |
| Isononyl Isononanoate | Dermol 99 | 10.16 | www.alzointernational.com |
| Hydrogenated Jojoba Oil | Jojoba Butter LM | 2.15 | www.desertwhale.com |
| Hydrogenated Jojaba Oil | Jojoba Butter HM | 1.00 | www.desertwhale.com |
| C30-45 Alkyl Methicone (and) C30-45 Olefin | Dow Corning AMS-C30 Cosmetic Wax | 1.15 | www.dowcorning.com |
| Stearyl Dimethicone | Dow Corning 2503 Cosmetic Wax | 0.47 | www.dowcorning. com |
| Cyclopentasiloxane (and) Polypropylsilsesquioxane | Dow Corning 670 Fluid | 5.00 | www.dowcorning.com |
| B | | | |
| Dimethicone/Vinyl Dimethicone Crosspolymer | Dow Corning 9506 Powder | 16.02 | www.dowcorning.com |
| Silica Dimethyl Silylate | Covasilic 15 | 0.17 | www.lcw.fr |
| Talc | Talc Powder | 5.00 | www.riedeldehaen.com |
| Mica (and) Titanium Dioxide (and) Iron Oxides | Prestige® Soft Belge | 5.00 | www.eckart.net |
| **Effektpigment nach Beispiel 2/3** | ----------------- | **0.05-10.00** | |
| C | | | |

(fortgesetzt)

| INCI Name | Product Name | %W/W | Designated use |
|---|---|---|---|
| Propylene Glycol, Diazolidinyl Urea, Methylparaben, Propylparaben | Germaben II | 0.40 | www.ispcorp.com |

Zubereitung:

1. Phase A mischen, Erhitzen bis alles geschmolzen ist

2. Phase B mit Speed Mixer vormischen (2400 rpm, 1 min)

3. Die Hälfte der geschmolzenen Phase A zu Phase B geben, im Speed Mixer mischen (2400 rpm, 30 s)

4. Rest der Phase A zu Phase B geben und im Speed Mixer mischen (2400 rpm, 30 s)

5. Phase C zufügen, im Speed Mixer mischen (2400 rpm, 30 s), auf Raumtemperatur abkühlen lassen

**Beispiel 16: Nail Polish**

| INCI Name | Product Name | %W/W | Supplier |
|---|---|---|---|
| A | | 100.00 | |
| **Effektpigment nach Beispiel 2/ 3** | ----------------- | **0.05 - 3.50** | |
| B | | | |
| International Lacquers Nailpolish & Care Base 359 | Butylacetat (and) Ethylacetat (and) Nitrocellulose (and) Isopropyl Alcohol | ad 100 | www.internationallacquers.lu |

Zubereitung:

1. Unter Rühren Phasen A und B mischen

2. In geeignetes Gefäß abfüllen

**Beispiel 17: Pressed Eye Shadow**

| INCI Name | Product Name | %W/W | Supplier |
|---|---|---|---|
| A | | 100.00 | |
| Mica | Silk Mica | 17.00 | www.vwr.com |
| Boron Nitride | Softouch CCS 102 | 2.50 | www.advceramicscos.com |
| Talc | Talc Powder | ad 100 | www.riedeldehaen.com |
| Zinc Stearate | Kemilub EZ-V | 7.00 | www.undesa.com |
| Mica (and) Iron Oxides | Prestige® Bright Fire-red | 39.00 | www.eckart.net |
| **Effektpigment nach Beispiel 2/ 3** | ----------------- | **0.05 - 8.00** | |

(fortgesetzt)

| INCI Name | Product Name | %W/W | Supplier |
|---|---|---|---|
| B | | | |
| Dimethicone | Dow Corning® 200 Fluid 5 cst | 5.00 | www.dowcorning.com |
| Cyclomethicone (and) Dimethicone Crosspolymer | Dow Corning® 9040 Elastomer | 5.00 | www.dowcorning.com |

Zubereitung:

1. Phase A mischen

2. Phase B hinzufügen und homogenisieren

3. Lidschatten bei 150 bar für 30 min pressen

**Beispiel 18: Pressed Powder**

| INCI Name | Product Name | %W/W | Supplier |
|---|---|---|---|
| A | | 100.00 | |
| Talc | Talc Powder | ad 100 | www.riedeldehaen.com |
| Zinc Stearate | Kemilub EZ-V | 5.00 | www.undesa.com |
| Methylparaben, Propylparaben | Rokonsal SSH-1 | 0.30 | www.biochema.com |
| Polyethylene | Asensa CL 111 | 20.50 | www.honeywell.com |
| Silica | Syloblanc 34 | 1.50 | www.gracedavision.com |
| Mica (and) Titanium Dioxide | Prestige® Soft Silver | 6.00 | www.eckart.net |
| Mica (and) Titanium Dioxide (and) Iron Oxides | Prestige® Soft Beige | 11.00 | www.eckart.net |
| **Effektpigment nach Beispiel 2/ 3** | ------------------ | **0.05 - 8.00** | |
| B | | | |
| Octyl Palmitate | Liponate EHP | 3.00 | www.lipochemicals.com |

Zubereitung:

1. Phase A mischen

2. Phase B hinzufügen und homogenisieren

3. Lidschatten bei 150 bar für 30 min pressen

**Beispiel 19: Shower Gel**

| INCI Name | Product Name | %W/W | Supplier |
|---|---|---|---|
| A | | 100.00 | 100.00 |
| **Effektpigment nach Beispiel 2/ 3** | ------------------ | **0.01 - 1.00** | |

(fortgesetzt)

| INCI Name | Product Name | %W/W | Supplier |
|---|---|---|---|
| Water | Aqua | ad 100 | |
| Blue 1 (0.5% aqueous solution) | FD&C Blue No.1 | 0.10 | www.sunchemicals.com |
| Acrylates/ C10-30 Alkyl Acrylate Crosspolvmer | Carbopol ETD 2020 | 1.00 | www.noveon.com |
| Propylene Glycol | 1, 2-Propanediol | 1.00 | www.vwr.com |
| B | | | |
| TEA-Lauryl Sulfate | Texapon T 42 | 22.00 | www.cognis.com |
| Cocamide Dea | Rewomid DC 212 S | 3.00 | www.degussa.com |
| Cocamidopropyl Betaine | Tego Betain F 50 | 4.00 | www.cognis.com |
| Disodium EDTA | Edeta BD | 0.05 | www.basf.com |
| C | | | |
| Triethanolamine | Triethanolamine | 0.30 | www.vwr.com |
| Phenoxyethanol, Ethylhexylglycerin | Euxyl PE 9010 | 0.60 | www.schuelke-mayr.com |

Zubereitung:

1. Carbopol in Phase A dispergieren

2. Auf 65°C erhitzen

3. Ingredientien aus Phase B nach und nach zugeben

4. Unter Rühren abkühlen lassen, bei 40-45°C Phase C zugeben

**Beispiel 20: Styling Soft Wax**

| INCI Name | Product Name | %W/W | Supplier |
|---|---|---|---|
| A | | 100.00 | |
| **Effektpigment nach Beispiel 2/ 3** | ------------------ | **0.01 - 2.00** | |
| Water | Aqua | ad 100 | |
| Propylene Glycol | 1,2 Propanediol | 2.00 | www.vwr.com |
| Glycerin | Pricerine 9090 | 7.00 | www.uniqema.com |
| Methoxy PEG/PPG-7/3 Aminopropyl Dimethicone | Abil Soft AF 100 | 0.50 | www.degussa.com |
| B | | | |
| Isosteareth-20 | Procol IS-20 | 14.50 | www.protameen.com |
| Laureth-4 | Genapol LA 040 | 10.00 | www.clariant.com |
| Paraffinum Liquidium | Paraffinum Liquidium | 6.00 | www.heess.de |
| C12-15 Alkyl Benzoate | Sympatens-LBZ | 6.00 | www.kolb.ch |
| C | | | |

(fortgesetzt)

| INCI Name | Product Name | %W/W | Supplier |
|---|---|---|---|
| Phenoxyethanol (and) Methylparaben (and) Butylparaben (and) Ethylparaben (and) Propylparaben (and) Isobutylparaben | Phenonip | 0.40 | www.clariant.com |
| Fragrance | Cool Floral OA D | 0.10 | www.bell-europe.com |

Zubereitung:

1. Phasen A und B separat auf 90°C erhitzen

2. Phase B unter Rühren zu Phase A hinzufügen

3. Auf 55°C abkühlen lassen

4. Phase C hinzufügen und in ein geeignetes Gefäß abfüllen

**Beispiel 21: Sun Protection Cream**

| INCI Name | Product Name | %W/W | Supplier |
|---|---|---|---|
| A | | 100.00 | |
| Sorbitan Stearate (and) Methyl Glucose Sesquistearte | Sympatens-O/2500 G | 5.00 | www.kolb.ch |
| Stearic Acid | Kortacid 1895 | 4.00 | www.akzonobel.com |
| Octyldodecanol | Eutanol G | 9.00 | www.cognis.com |
| Caprylic/ Capric Triglyceride | Liponate GC-K | 10.00 | www.lipochemicals.com |
| Cetearyl Alcohol | Lanette O | 2.00 | www.cognis.com |
| Macadamia ternifolia Seed Oil | Macadamia Nut Oil | 3.20 | www.jandekker.com |
| Octyl Methoxycinnamate | Parsol MCX | 1.00 | www.dsm.com |
| Butyl Methoxydibenzoylmethane | Parsol 1789 | 5.00 | www.roche.com |
| B | | | |
| **Effektpigment nach Beispiel 2/3** | ----------------- | **0.05 - 3.00** | |
| Water | Aqua | ad 100 | |
| Glycerin | Pricerine 9090 | 3.20 | www.uniqema.com |
| C | | | |
| Phenoxyethanol (and) Methylparaben (and) Ethylparaben (and) Butylparaben | Uniphen P-23 | 0.40 | www.induchem.com |
| Fragrance | Nivamar BM | 0.15 | www.bell-europe.com |

Zubereitung:

1. Phasen A und B separat auf 80°C erhitzen

2. Phase A unter Rühren zu Phase B hinzufügen

3. Auf 45°C abkühlen lassen

4. Phase C hinzufügen

**Beispiel 22: Transparent Lipstick**

| INCI Name | Product Name | %W/W | Supplier |
|---|---|---|---|
| A | | 100.00 | |
| Ethylenediamine/ Hydrogenated Dimer Dilinoleate Copolymer Bis-Di-C14-18 Alkyl Amide | Sylvaclear A2614V | 28.00 | www.arizonachemical.com |
| Bis-Stearyl Ethylenediamine/ Neopentyl Glycol/ Hydrogenated Dimer Dilinoleate | Sylvaclear C75V | 28.00 | www.arizonachemical.com |
| Paraffinum Liquidum | Paraffinum Liquidum | 13.80 | www.heess.de |
| Macadamia Integrifolia Seed Oil | Floramac Hawaiian Macadamia Oil-Refined | ad 100 | www.floratech.com |
| Isopropyl Myristate | Isopropyl Myristate | 6.00 | www.vwr.com |
| C12-15 Alkyl Benzoate | Sympatens-LBZ | 6.00 | www.kolb.ch |
| Caprylic/ Capric Triglyceride | Miglyol 812 | 7.00 | www.sasolwax.com |
| Propylparaben | Propyl-4-hydroxybenzoat | 0.20 | www.sigmaaldrich.com |
| B | | | |
| **Effektpigment nach Beispiel 2/3** | ----------------- | **0.01 - 3.50** | |

Zubereitung:

1. Phase A auf 85°C erhitzen

2. Phase B zu Phase A hinzufügen und Vermischen

3. Bei 75°C in eine Lippenstiftform abfüllen

**Beispiel 23: Body Lotion Water-In-Silicone**

| INCI Name | Product Name | %W/W | Supplier |
|---|---|---|---|
| A | | 100.00 | |
| Cyclopentasiloxane (and) Dimethiconol | Dow Corning 1501 | 11.50 | www.dowcorning.com |
| Cyclopentasiloxane | Dow Corning 245 | 5.75 | www.dowcorning.com |
| Cyclopentasiloxane (and) PEG/PPG/- 18/18 Dimethicone | Dow Corning 5225 C | 13.80 | www.dowcorning.com |
| C 30-45 Alkyl Methicone | Dow Corning Cosmetic Wax AMS-C30 | 3.45 | www.dowcorning.com |

(fortgesetzt)

| INCI Name | Product Name | %W/W | Supplier |
|---|---|---|---|
| **Effektpigment nach Beispiel 2/3** | ------------------ | **0.05 - 3.50** | |
| C | | | |
| Polysorbate 20 | Tween 20 | 0.60 | www.uniqema.com |
| Phenoxyethanol (and) Methylparaben (and) Ethylparaben (and) Butylparaben | Uniphen P-23 | 0.35 | www.induchem.com |
| Sodium Chloride | Sodium Chloride | 0.75 | www.vwr.com |
| Water | Aqua | ad 100 | |

Zubereitung:

1. Phase A mischen und auf 75°C erhitzen

2. Phase B mischen und auf 70°C erhitzen

3. Phase B langsam unter Homogenisieren zu Phase A hinzufügen und unter Rühren abkühlen lassen

**Beispiel 24: Tinted Day Cream**

| INCI Name | Product Name | %W/W | Supplier |
|---|---|---|---|
| A | | 100.00 | |
| Mica (and) Titanium Dioxide (and) Iron Oxides | Prestige®Soft Beige | 1.00 | www.eckart.net |
| Mica (and) Titanium Dioxide | Prestige®Soft Red | 2.00 | www.eckart.net |
| **Effektpigment nach Beispiel 2/ 3** | ------------------ | **0.05 - 3.00** | |
| Silica | Syloblanc 34 | 3.00 | www. gracedavision.com |
| Glycerin | Pricerine 9090 | 5.00 | www.uniqema.com |
| Allantoin | Allantoin | 0.40 | www.3V.com |
| Mica | Silk Mica | 3.00 | www.vwr.com |
| Boron Nitride | Boroneige #1501 | 1.50 | www.esk.com |
| Water | | ad 100 | |
| Xanthan Gum | Keltrol T | 0.20 | www.cpkelco.com |
| B | | | |
| Glyceryl Stearate (and) PEG-100 Stearate | Lipomulse 165 | 2.00 | www.lipochemicals.com |
| Ceteryl Alcohol (and) Cetearyl Glucoside | Montanov 68 | 2.20 | www.seppic.com |
| Dimethicone | Dow Coming 200 Fluid/ 350 cst | 1.00 | www.dowcorning.com |
| Cetearyl Isononanoate | Tego Soft Cl | 5.10 | www.degussa.com |

(fortgesetzt)

| INCI Name | Product Name | %W/W | Supplier |
|---|---|---|---|
| Octyldodecanol | Eutanol G | 4.00 | www.cognis.com |
| Butyrospermum Parkii (Shea Butter) | Shea Butter | 3.80 | www.jandekker.com |
| C | | | |
| Phenoxyethanol (and) Methylparaben (and) Ethylparaben (and) Butylparaben | Uniphen-23 | 0.40 | www.permcos.com |
| Fragrance | Sensuality | 0.10 | www.drom.de |

Zubereitung:

1. Phase A mischen, Keltrol dispergieren

2. Auf 80°C erhitzen

3. Phase B auf 80°C erhitzen

4. Phase B langsam zu Phase A hinzugeben

5. Auf 40°C abkühlen lassen und Phase C hinzufügen

**Beispiel 25: Hair Mascara**

| INCI Name | Product Name | %W/W | Supplier |
|---|---|---|---|
| A | | 100.00 | |
| Polyquaternium-16 | Luviquat FC 905 (Luviquat Exellence) | 2.70 | www.basf.com |
| Propylene Glycol | 1,2-Propanediol | 1.80 | www.vwr.com |
| Methylparaben | Methyl-4-hydroxybenzoate | 0.20 | www.sigmaaldrich.com |
| Aqua | Water | ad 100 | |
| B | | | |
| Cetearyl Alcohol | Lanette O | 5.00 | www.cognis.com |
| Dimethicone | Dow Corning 200 Fluid/ 350 cst | 1.00 | www.dowcorning.com |
| Ceteareth-25 | Cremophor A 25 | 2.00 | www.basf.com |
| Propylparaben | Propyl-4-hydroxybenzoat | 0.10 | www.sigmaaldrich.com |
| C | | | |
| Hydroxypropylcellulose | Klucel G | 0.50 | www.herc.com |
| Magnesium Aluminium Silicate | Veegum HV | 0.50 | www.rtvanderbilt.com |
| Aqua | Water | 19.00 | |
| D | | | |

(fortgesetzt)

| INCI Name | Product Name | %W/W | Supplier |
|---|---|---|---|
| **Effektpigment nach Beispiel 2/ 3** | ------------------ | **0.10 - 8.00** | |
| Phenoxyethanol (and) Methylparaben (and) Butylparaben (and) Ethylparaben (and) Propylparaben (and) Isobutylparaben | Phenonip | 0.20 | www.clariant.com |
| Fragrance | Blue Shadow ÖKO | 0.05 | www.bell-europe.com |

Zubereitung:

    1. Phasen A und B separat auf 80°C erhitzen

    2. Phase B langsam zu Phase A hinzugeben

    3. In separatem Gefäß Klucel und Veegum in das Wasser der Phase C einrühren

    4. Phase AB auf 40°C abkühlen lassen

    5. Phasen C und D hinzufügen

**Beispiel 26: Perfume Powder Stick**

| INCI Name | Product Name | %W/W | Supplier |
|---|---|---|---|
| A | | 100.00 | |
| Dimethicone/ Vinyl Dimethicone Crosspolymer and Silica | Dow Corning 9701 Cosmetic Powder | ad 100 | www.dowcorning.com |
| Talc | Talc Powder | 29.00 | www.riedeldehaen.com |
| Amorphoeus Silica | Spheron P-1500 | 1.00 | www.cheshamchemicals.co.uk |
| **Effektpigment nach Beispiel 2/ 3** | | **0.06 - 8.00** | |
| | ------------------ | | |
| Fragrance | Moisturadiance | 5.00 | www.drom.com |

Zubereitung:

    1. Phase A im Speedmixer mischen (30 s/ 2000 rpm)

    2. Phase B zu Phase A hinzufügen und im Speed Mixer mischen (30 s/ 2500 rpm)

    3. In geeignetes Gefäß abfüllen

**Beispiel 27: Pressed Blush**

| INCI Name | Product Name | %W/W | Designated use |
|---|---|---|---|
| A | | 100.00 | |
| Mica | Silk Mica | ad 100 | www.vwr.com |
| Nylon 12 | Orgasol 2002D/ Nat | 3.20 | www.atofinachemicals.com |
| Boron Nitride | Boroneige #1501 | 3.00 | www.esk.com |
| Talc | Talc Powder | 15.00 | www.riedeldehaen.com |
| Zinc Stearate | Kemilub EZ-V | 2.00 | www.undesa.com |
| **Effektpigment nach Beispiel 2/ 3** | ------------------ | **0.05 - 8.00** | |
| | | | |
| Octyl Palmitate | Liponate EHP | 8.00 | www.lipochemicals.com |

Zubereitung:

1. Phase A mischen

2. Phase B hinzufügen und homogenisieren

3. Blush bei 150 bar 30s pressen

**Patentansprüche**

**1.** Effektpigmente, umfassend künstliche plättchenförmige Substrate, die mindestens eine optisch wirksame Beschichtung aufweisen,
**dadurch gekennzeichnet,**
**dass** die Effektpigmente eine volumengemittelte Größensummendurchgangsverteilungskurve mit den Kennzahlen $D_{10}$, $D_{50}$ und $D_{90}$ aufweisen, wobei diese Größensummendurchgangsverteilungskurve einen Span $\Delta D$ von 0,7 -1,4 aufweist und der Span $\Delta D$ gemäß Formel (I) berechnet ist:

$$\Delta D = (D_{90} - D_{10})/ D_{50}, \qquad\qquad (I)$$

und wobei die mittlere Dicke der künstlichen plättchenförmigen Substrate 500 nm bis 2.000 nm beträgt und wobei die $D_{10}$-, $D_{50}$- und $D_{90}$-Werte aus den folgenden Kombinationen von Werterbereichen:

| $D_{90}$ ($\mu$m) | $D_{50}$ ($\mu$m) | $D_{10}$ ($\mu$m) |
|---|---|---|
| 20 - 45 | 10 - 35 | 5 - 25 |
| 70 - 110 | 30 - 65 | 20 - 45 |
| 120 - 180 | 40 - 140 | 25 - 65 |
| 400 - 490 | 135 - 250 | 75 - 110 |

ausgewählt werden mit der Maßgabe, dass die gewählte Kombination der $D_{10}$-, $D_{50}$- und $D_{90}$-Werte einen Span $\Delta D$ von 0,7 bis 1,4 ergeben.

**2.** Effektpigmente nach Anspruch 1,
**dadurch gekennzeichnet,**
**dass** die mittlere Dicke der künstlichen plättchenförmigen Substrate 750 nm bis 1.500 nm beträgt.

3. Effektpigmente nach einem der vorangehenden Ansprüche,
**dadurch gekennzeichnet,**
**dass** die Standardabweichung der Dicke der künstlichen Substrate 15 % bis 100 % beträgt.

4. Effektpigmente nach einem der vorangehenden Ansprüche,
**dadurch gekennzeichnet,**
**dass** die Effektpigmente einen Span $\Delta D$ von 0,8 - 1,1 aufweisen.

5. Effektpigmente nach einem der vorangehenden Ansprüche,
**dadurch gekennzeichnet,**
**dass** wobei die Effektpigmente zusätzlich zu der wenigstens einen optisch wirksamen Beschichtung eine äußere Schutzschicht aufweisen.

6. Effektpigmente nach Anspruch 5,
**dadurch gekennzeichnet,**
**dass** die äußere Schutzschicht eine oder zwei Metalloxidschichten der Elemente Si, Al oder Ce umfasst.

7. Effektpigmente nach einem der Ansprüche 5 oder 6,
**dadurch gekennzeichnet,**
**dass** die äußere Schutzschicht an der Oberfläche organisch-chemisch modifiziert ist.

8. Effektpigmente nach einem der Ansprüche 5 bis 7,
**dadurch gekennzeichnet,**
**dass** ein oder mehrere Silane auf der äußeren Schutzschicht aufgebracht sind.

9. Effektpigmente nach einem der Ansprüche 5 bis 8,
**dadurch gekennzeichnet,**
**dass** die Silane wenigstens ein Silan ohne funktionelle Bindungsgruppe und wenigstens ein aminofunktionelles Silan enthalten.

10. Effektpigmente nach einem der vorangehenden Ansprüche,
**dadurch gekennzeichnet,**
**dass** die mindestens eine optisch wirksame Beschichtung einen Brechungsindex von $n \geq 1,9$ aufweist und aus der Gruppe, die aus Metalloxid, Metallhydroxid, Metalloxidhydrat, Metallsuboxid und Mischungen davon besteht, ausgewählt wird.

11. Effektpigmente nach Anspruch 10,
**dadurch gekennzeichnet,**
**dass** die Effektpigmente in einem Rakelabzug eines Lackes, enthaltend 6 Gew.-% Effektpigmente in einem farblosen Nitrozelluloselack, bezogen auf das Gesamtgewicht des Lackes, mit 76 $\mu$m Naßfilmdicke nach dessen Trocknung ein 90% Quantil der Farbabstandsverteilung $\Delta C^*_{90}$ im a*-b*-Farbraum von 0,5 bis 8,0 aufweisen.

12. Effektpigmente nach einem der vorangehenden Ansprüche,
**dadurch gekennzeichnet,**
**dass** die Größensummendurchgangsverteilungskurve einen $D_{50}$-Wert in einem Bereich von 10 bis 300 $\mu$m aufweist.

13. Effektpigmente nach einem der vorangehenden Ansprüche,
**dadurch gekennzeichnet,**
**dass** die künstlichen Substrate im wesentlichen transparent sind und vorzugsweise aus der Gruppe, bestehend aus Glasplättchen, Plättchen aus synthetischem Glimmer, $SiO_2$-Plättchen, Polymerplättchen, plättchenförmigem Bismuthoxychlorid, plättchenförmigen Aluminiumoxiden und Gemischen davon, ausgewählt werden.

14. Effektpigmente nach einem der vorangehenden Ansprüche,
**dadurch gekennzeichnet,**
**dass** die künstlichen Substrate aus der Gruppe, bestehend aus Glasplättchen, Plättchen aus synthetischem Glimmer und Gemischen davon, ausgewählt werden.

15. Verwendung der Effektpigmente nach einem der Ansprüche 1 bis 14 in Kosmetika, Kunststoffen und Beschichtungszusammensetzungen wie Farben, Druckfarben, Lacken, Pulverlacken, Elektrotauchlacken.

**Claims**

1. Effect pigments, comprising artificial platelet-shaped substrates having at least one optically active coating, **characterised in that**
the effect pigments have a volume-averaged, cumulative undersize distribution curve with the characteristic numbers $D_{10}$, $D_{50}$ and $D_{90}$, and this cumulative undersize distribution curve has a span $\Delta D$ of 0.7 - 1.4, and the span $\Delta D$ is calculated on the basis of formula (I):

$$\Delta D = (D_{90} - D_{10})/D_{50} \qquad (I)$$

and the mean thickness of the artificial platelet-shaped substrates is 500 nm to 2,000 nm and the $D_{10}$, $D_{50}$ and $D_{90}$ values are selected from the following combinations of value ranges:

| $D_{90}$ ($\mu$m) | $D_{50}$ ($\mu$m) | $D_{10}$ ($\mu$m) |
|---|---|---|
| 20 - 45 | 10 - 35 | 5 - 25 |
| 70 - 110 | 30 - 65 | 20 - 45 |
| 120 - 180 | 40 - 140 | 25 - 65 |
| 400 - 490 | 135 - 250 | 75 - 110 |

provided the selected combination of $D_{10}$, $D_{50}$ and $D_{90}$ values produces a span $\Delta D$ of 0.7 to 1.4.

2. Effect pigments as claimed in claim 1,
**characterised in that**
the average thickness of the artificial platelet-shaped substrates is 750 nm to 1,500 nm.

3. Effect pigments as claimed in one of the preceding claims,
**characterised in that**
the standard variance of the thickness of the artificial substrates is 15% to 100%.

4. Effect pigments as claimed in one of the preceding claims,
**characterised in that**
the effect pigments have a span $\Delta D$ of 0.8 - 1.1.

5. Effect pigments as claimed in one of the preceding claims,
**characterised in that**
in addition to the at least one optically active coating, the effect pigments have an outer protective layer.

6. Effect pigments as claimed in claim 5,
**characterised in that**
the outer protective layer comprises one or two metal oxide layers of the elements Si, Al or Ce.

7. Effect pigments as claimed in one of claims 5 or 6,
**characterised in that**
the outer protective layer is organic-chemically modified on the surface.

8. Effect pigments as claimed in one of claims 5 to 7,
**characterised in that**
one or more silanes are applied to the outer protective layer.

9. Effect pigments as claimed in one of claims 5 to 8,
**characterised in that**
the silanes contain at least one silane without a functional binding group and at least one amino functional silane.

10. Effect pigments as claimed in one of the preceding claims, **characterised in that** the at least one optically active coating has a refractive index of n ≥ 1.9 and is selected from the group comprising metal oxide, metal hydroxide, metal oxide hydrate, metal sub-oxide and mixtures thereof.

11. Effect pigments as claimed in claim 10, **characterised in that** in an applicator drawdown of a varnish containing 6 % by weight of effect pigments in a colourless nitro-cellulose varnish, by reference to the total weight of the varnish applied with a 76 $\mu$m wet film thickness after drying, the effect pigments have a 90 % quantile of the colour difference distribution $\Delta C*90$ in the a*-b*-colour space of 0.5 to 8.0.

12. Effect pigments as claimed in one of the preceding claims, **characterised in that** the cumulative undersize distribution curve has a $D_{50}$ value in a range of 10 to 300 $\mu$m.

13. Effect pigments as claimed in one of the preceding claims, **characterised in that** the artificial substrates are substantially transparent and are preferably selected from the group comprising glass platelets, platelets of synthetic mica, $SiO_2$ platelets, polymer platelets, platelet-shaped bismuth oxychloride, platelet-shaped aluminium oxides and mixtures thereof.

14. Effect pigments as claimed in one of the preceding claims, **characterised in that** the artificial substrates are selected from the group comprising glass platelets, platelets of synthetic mica and mixtures thereof.

15. Use of the effect pigments as claimed in one of claims 1 to 14 in cosmetics, plastics and coating compositions, such as paints, printing inks, varnishes, powder coatings and electro-dip coatings.

**Revendications**

1. Pigments à effets, comprenant des substrats lamellaires artificiels qui présentent au moins un revêtement optiquement actif, **caractérisés en ce que** les pigments à effets présentent une courbe de distribution cumulée en volume ayant les caractéristiques $D_{10}$, $D_{50}$ et $D_{90}$, cette courbe de distribution cumulée en volume présentant une polydispersité $\Delta D$ de 0,7 à 1,4, et la polydispersité $\Delta D$ étant calculée selon la formule (I) :

$$\Delta D = (D_{90} - D_{10})/D_{50} \qquad (I)$$

et dans lesquels l'épaisseur moyenne des substrats lamellaires artificiels est de 500 nm à 2000 nm, et dans lesquels les valeurs de $D_{10}$, $D_{50}$ et $D_{90}$ sont choisies parmi les combinaisons suivantes de plages de valeurs :

| $D_{90}$ ($\mu$m) | $D_{50}$ ($\mu$m) | $D_{10}$ ($\mu$m) |
|---|---|---|
| 20 - 45 | 10 - 35 | 5 - 25 |
| 70 - 110 | 30 - 65 | 20 - 45 |
| 120 - 180 | 40 - 140 | 25 - 65 |
| 400 - 490 | 135 - 250 | 75 - 110 |

à la condition que la combinaison choisie des valeurs de $D_{10}$, $D_{50}$ et $D_{90}$ conduise à une polydispersité $\Delta D$ de 0,7 à 1,4.

2. Pigments à effets selon la revendication 1, **caractérisés en ce que** l'épaisseur moyenne des substrats lamellaires artificiels est de 750 nm à 1500 nm.

3. Pigments à effets selon l'une des revendications précédentes, **caractérisés en ce que** l'écart-type de l'épaisseur des substrats artificiels et de 15 % à 100 %.

**4.** Pigments à effets selon l'une des revendications précédentes, **caractérisés en ce que** les pigments à effets présentent une polydispersité $\Delta D$ de 0,8 à 1,1.

**5.** Pigments à effets selon l'une des revendications précédentes, **caractérisés en ce que** les pigments à effets présentent, en plus de l'au moins un revêtement optiquement actif, une couche de protection extérieure.

**6.** Pigments à effets selon la revendication 5, **caractérisés en ce que** la couche de protection extérieure comprend un ou deux couches d'oxyde métallique des éléments S, Al ou Ce.

**7.** Pigments à effets selon l'une des revendications 5 ou 6, **caractérisés en ce que** la couche de protection extérieure a subi sur sa surface une modification organique-chimique.

**8.** Pigments à effets selon l'une des revendications 5 à 7, **caractérisés en ce qu'**un ou plusieurs silanes sont appliqués sur la couche de protection extérieure.

**9.** Pigments à effets selon l'une des revendications 5 à 8, **caractérisés en ce que** les silanes contiennent au moins un silane sans groupe de liaison fonctionnel et au moins un silane à fonctionnalité amino.

**10.** Pigments à effets selon l'une des revendications précédentes, **caractérisés en ce que** l'au moins un revêtement optiquement actif présente un indice de réfraction $n \geq 1,9$ et est choisi dans le groupe constitué d'un oxyde métallique, d'un hydroxyde métallique, d'un oxyde métallique hydraté, d'un sous-oxyde métallique et de mélanges de ceux-ci.

**11.** Pigments à effets selon la revendication 10, **caractérisés en ce que** les pigments à effets présentent, dans une raclure d'un vernis contenant 6 % en poids de pigments à effets dans un vernis de nitrocellulose incolore, et par rapport au poids total du vernis, présentant une épaisseur de feuil humide de 76 $\mu$m après son séchage, un quantile à 90 % de la distribution des différences de couleur $\Delta C^*_{90}$, dans l'espace colorimétrique a*-b*, de 0,5 à 8,0.

**12.** Pigments à effets selon l'une des revendications précédentes, **caractérisés en ce que** la courbe de distribution cumulée présente une valeur de $D_{50}$ comprise dans la plage de 10 à 300 $\mu$m.

**13.** Pigments à effets selon l'une des revendications précédentes, **caractérisés en ce que** les substrats artificiels sont pour l'essentiel transparents et sont de préférence choisis dans le groupe consistant en les lamelles de verre, les lamelles de mica synthétique, des lamelles de $SiO_2$, les lamelles polymères, l'oxychlorure de bismuth lamellaire, les oxydes d'aluminium lamellaires et les mélanges de ceux-ci.

**14.** Pigments à effets selon l'une des revendications précédentes, **caractérisés en ce que** les substrats artificiels sont choisis dans le groupe consistant en les lamelles de verre, les lamelles de mica synthétique et les mélanges de ceux-ci.

**15.** Utilisation des pigments à effets selon l'une des revendications 1 à 14 dans des produits cosmétiques, des matières plastiques et des compositions de revêtement telles que des peintures, des encres d'imprimerie, des vernis, des peintures en poudre et des peintures électrophorétiques.

Abbildung 1:

Abbildung 2:

Verteilung der Farborte im a*b*-Farbraum

Abbildung 3:

$\Delta C*^{90}$ der Farbabstandsverteilung

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- US 3331699 A
- US 5436077 A
- US 6045914 A
- WO 2004055119 A1
- WO 2002090448 A2
- WO 2006110359 A2
- WO 2007114442 A1
- US 20060042509 A1
- EP 2009066 A1
- WO 9729059 A1
- US 5156889 A
- WO 2006021386 A1